# EUROPEAN PATENT SPECIFICATION

(11) **EP 1 556 513 B1**
(45) Date of publication and mention of the grant of the patent: **28.04.2010**
(21) Application number: 03776419.8
(22) Date of filing: 16.10.2003
(51) Int. Cl.: A61K 39/12, C12N 15/85

(54) **COMPOSITIONS AND METHODS FOR TREATING HUMAN PAPILLOMAVIRUS-MEDIATED DISEASE**
ZUSAMMENSETZUNGEN UND VERFAHREN ZURBEHANDLUNG VON KRANKHEITEN, DIE DURCH DAS HUMANE PAPILLOMAVIRUS VERMITTELTWERDEN
COMPOSITIONS ET METHODES POUR TRAITER UNE MALADIE A MEDIATION PAR LE PAPILLOMAVIRUS HUMAIN (HPV)

(30) Priority: 21.10.2002 US 420068 P; 15.05.2003 US 471481 P
(43) Date of publication of application: 27.07.2005
(73) Proprietor: Eisai Inc., Woodcliff Lake, NJ 07677 (US)
(72) Inventor: BEACH, Kathleen, Amherst, MA 01002 (US); HEDLEY, Mary, Lynne, Lexington, MA 02421 (US); URBAN, Robert, G., Lexington, MA 02421 (US); CHICZ, Roman M., Belmont, MA 02478 (US)
(74) Representative: Dörries, Hans Ulrich
(86) International application number: PCT/US2003/032705
(87) International publication number: WO 2004/037175

(56) References cited:
- WO-A1-01/19408
- US-B1- 6 306 397
- KLENCKE B ET AL: "Encapsulated plasmid DNA treatment for human papillomavirus 16-associated anal dysplasia: a phase I study of ZYC101" CLINICAL CANCER RESEARCH, THE AMERICAN ASSOCIATION FOR CANCER RESEARCH, US, vol. 8, May 2002 (2002-05), pages 1028-1037, XP002977551 ISSN: 1078-0432
- SHEETS E.E. ET AL.: "Immunotherapy of human cervical high-grade cervical intraepithelial neoplasia with microparticle-delivered human papillomavirus 16 E7 plasmid DNA" AM. J. OBSTET. GYNECOL., vol. 188, April 2003 (2003-04), pages 916-926, XP002417614
- GARCIA F. ET AL.: "ZYC101a for treatment of high-grade cervical intraepithelial neoplasia: a randomized controlled trial." OBSTET GYNECOL, vol. 103, 2004, pages 317-326, XP002417615
- KLENCKE B. ET AL.: 'Encapsulated plasmid DNA treatment for human papillomavirus 16-associated anal dysplasia: a phase I study of ZYC101' CLINICAL CANCER RESEARCH vol. 8, May 2002, pages 1028 - 1037, XP002977551
- MOSCICKI A-B.: 'Human papillomavirus infection in adolescents' ADOLESCENT GYNECOLOGY, PART II vol. 46, no. 4, August 1999, pages 783 - 807, XP002977552
- HEDLEY M.L. ET AL.: 'Microspheres containing plasmid-encoded antigens elicit cytotoxic T-cell responses' NATURE MEDICINE vol. 4, no. 3, March 1998, pages 365 - 368, XP002160611

## Description

### Field of the Invention

This invention relates to compositions for treating cevical intraepithelial neoplasia (CIN).

### Background of the Invention

Human papillomaviruses (HPV) are some of the most commonly sexually transmitted pathogens in the United States. Over 100 types of HPV have been isolated and categorized as either cutaneous HPV or mucosal HPV. Cutaneous HPVs include more than 15 types of HPVs that are associated with different types of skin warts. Mucosal HPVs include more than 25 types of HPVs and are mainly found in the genital tract, respiratory tract, and oral cavity.

Certain types of HPV cause benign warts, or papillomas, that persist for several months to years. In some cases, the growth of warts may become life-threatening, for example, in the respiratory tract. In other cases, warts cause discomfort, pain, hoarseness of voice, perceived cosmetic flaws and may serve as a source of virus for sexual transmission of HPV.

At least 18 HPV subtypes (e.g., HPV-16, HPV-18, HPV-31, HPV-33, HPV-35, HPV-39, HPV-45, HPV-51, HPV-52, HPV-56, HPV-58, HPV-59, HPV-68, HPV-73, HPV-82, HPV-26, HPV-53, and HPV-66) are associated with the development of cervical cancer (Munoz et al NEJM 348:6, 2003). Cancer of the uterine cervix is the second most common cancer among women worldwide. Annually, approximately 450,000 new cases are diagnosed and almost 200,000 deaths are due to cervical cancer (Pisani, et al., Int. J. Cancer 55:891-903, 1993). The overall 5-year survival rate is about 60%. However, survival rates range from 15%, to 85%, depending on how advanced the cancer is when the patient is diagnosed (Murakami et al., J. Immunother. 22(3):212-218, 1999).

The relationship between persistent HPV infection and cancer has focused attention on medical approaches for the treatment of precancerous lesions, specifically intraepithelial neoplasia before progression to invasive disease. Currently available means of therapy for cervical intraepithelial neoplasia include ablative or surgical procedures that remove the diseased areas. In the case of cervical intraepithelial neoplasia, ablation is typically performed by cryotherapy and the standard surgical procedure is Loop electrosurgical excision procedure (LEEP). Although these treatments carry a high cure rate, they have significant disadvantages including local surgical complications, cervical scarring which confounds future diagnoses, and the risk of complications to childbearing. In addition, these treatment options do not prevent the recurrence of precancerous lesions or protect from re-infection that is a result of continued sexual activity.

More recently vaccination strategies have been discussed for treating HPV-mediated diseases.

One example for such therapeutic vaccine is ZYC101, a plasmid DNA encoding multiple HLA-A2-restricted epitopes derived from the HPV-16 E7 protein (Klenck et al., Clinical Cancer Research 8: 1028-1037, 2002).

Further, ZYC101a has been described for treating a variety of HPV-mediated diseases, among others cervical intraepithelial neoplasia (WO 01/19408).

### Summary of the Invention

The invention is based, at least in part, on the discovery that a pharmaceutical composition containing a nucleic acid that encodes a polypeptide containing an epitope of a naturally occurring HPV protein is highly effective in treating HPV-mediated disease in persons within a specific range of ages.

The invention is also based, at least in part, on the discovery that a pharmaceutical composition containing a nucleic acid that encodes a polypeptide containing an epitope of a naturally occurring first type of HPV protein elicits a cross-reactive immune response that provides a therapeutic benefit for an infection with a second type of HPV

In one aspect, the present invention relates to the use of a nucleic acid comprising a nucleotide sequence that encodes a hybrid polypeptide for the preparation of a pharmaceutical composition for treating a cervical intraepithelial neoplasia (CIN) in a human identified as being less than 25 years of age, wherein the hybrid polypeptide comprises
at least one of the following segments of human papilloma virus (HPV) strain 16 E6: AMFQDPQERPRKLPQLCTEL,
LLRREVYDFAFRDLCIVYRDGNPY, or
KISEYRHYCYSLYGTTLEQQYNK;
at least one of the following segments of HPV strain 16 E7: TLHEYMLDLQPETTDLYSY,
QAEPDRAHYNIVTF, or
LLMGTLGIVCPICSQKP;
at least one of the following segments of HPV strain 18 E6:
RRPYKLPDLCTELNTSLQDIEITCVYCKTVLELTEVFEFAFK, or
SVYGDTLEKLTNTGLYNLLIRCLRCQK; and
at least one of the following segments of HPV strain 18 E7:
KATLQDIVLHLEPQNEIPV,
HTMLCMCCKCEARI, or
AFQQLFLNTLSFVCPWC.

In another aspect, the invention relates to a composition comprising a nucleic acid comprising a nucleotide sequence that encodes a hybrid polypeptide for use in treating a cervical intraepithelial neoplasia (CIN) in a human identified as being less than 25 years of age, wherein the hybrid polypeptide comprises
at least one of the following segments of human papilloma virus (HPV) strain 16 E6:
AMFQDPQERPRKLPQLCTEL,
LLRREVYDFAFRDLCIVYRDGNPY, or
KISEYRHYCYSLYGTTLEQQYNK;
at least one of the following segments of HPV strain 16 E7:
TLHEYMLDLQPETTDLYSY,
QAEPDRAHYNIVTF, or
LLMGTLGIVCPICSQKP;
at least one of the following segments of HPV strain 18 E6:
RRPYKLPDLCTELNTSLQDIEITCVYCKTVLELTEVFEFAFK, or
SVYGDTLEKLTNTGLYNLLIRCLRCQK; and
at least one of the following segments of HPV strain 18 E7:
KATLQDIVLHLEPQNEIPV,
HTMLCMCCKCEARI, or
AFQQLFLNTLSFVCPWC.

In a particular embodiment of the invention, the hybrid polypeptide comprises the segments AMFQDPQERPRKLPQLCTEL, LLRREVYDFAFRDLCIVYRDGNPY, KISEYRHYCYSLYGTTLEQQYNK, TLHEYMLDLQPETTDLYSY, QAEPDRAHYNIVTF, LLMGTLGIVCPICSQKP, RRPYKLPDLCTELNTSLQDIEITCVYCKTVLELTEVFEFAFK, SVYGDTLEKLTNTGLYNLLIRCLRCQK, KATLQDIVLHLEPQNEIPV, HTMLCMCCKCEARI, and AFQQLFLNTLSFVCPWC.

In another embodiment, the hybrid polypeptide does not contain a sequence identical to the sequence of either full length, intact E6 or full length, intact E7 protein from HPV strain 16 or 18.

In yet further embodiements, the hybrid polypeptide comprises a signal sequence. Preferably, the signal sequence is the HLA-DRα leader sequence (MAISGVPVLGFFIIAVLMSAQESWA). Thus, the hybrid polypeptide may comprises the amino acid sequence
AMFQDPQERPRKLPQLCTELLLRREVYDFAFRDLCIVYRDGNPYKISEYRHYCYSLYGTTLE QQYNKTLHEYMLDLQPETTDLYSYQAEPDRAHYNIVTFLLMGTLGIVCPICSQKPRRPYKLPD LCTELNTSLQDIEITCVYCKTVLELTEVFEFAFKSVYGDTLEKLTNTGLYNLLIRCLRCQKKATL QDIVLHLEPQNEIPVHTMLCMCCKCEARIAFQQLFLNTLSFVCPWC.
or may comprise or consist of the amino acid sequence MAISGVPVLGFFIIAVLMSAQESWAAMFQDPQERPRKLPQLCTELLLRREVYDFAFRDLCIVY RDGNPYKISEYRHYCYSLYGTTLEQQYNKTLHEYMLDLQPETTDLYSYQAEPDRAHYNIVTF LLMGTLGIVCPICSQKPRRPYKLPDLCTELNTSLQDIEITCVYCKTVLELTEVFEFAFKSVYGDT LEKLTNTGLYNLLIRCLRCQKKATLQDIVLHLEPQNEIPVHTMLCMCCKCEARIAFQQLFLNTL SFVCPWC.

The present invention therefore describes a pharmaceutical composition comprising a nucleic acid that encodes a hybrid polypeptide comprising epitopes of naturally occurring papillomavirus (HPV) proteins as defined above.

This pharmaceutical composition can be administered in an effective amount to a human of less than 25 years of age to treat cervical intraepithelial neoplasia (CIN). The composition can be used even if prior to the administration the individual is not tested to determine the identity of one or more types of HPV present in the individual. It can be used even if the individual is not infected with an HPV type that encodes a protein a portion of which is identical to an epitope present In the polypeptide encoded by the nucleic acid.

Also envisaged is a kit comprising said composition and instructions for its use In persons less than 25 years of age.

As used herein, the term "treatment" is defined as the administration of a pharmaceutical composition to an individual who has a disease or a symptom of a disease, with the effect of eliminating or reducing severity of the disease or one or more symptoms of the disease. For example, a composition for use in a treatment described herein can resolve a lesion, reduce or eliminate the need for an invasive procedure, prevent lesion recurrence, and/or prevent or delay progression of a lesion to carcinoma.

A "pharmaceutical composition" contains a therapeutically effective amount of a nucleic acid described herein as well as a pharmaceutically acceptable carrier (e.g., a diluent, adjuvant, excipient, or vehicle with which the nucleic acid is administered).

As used herein, the term "epitope" is defined as a peptide that binds to the binding groove of an MHC class I or class II molecule.

As used herein, "identified as being effective for treating an HPV-mediated disease (e.g., a CIN) in persons less than 25 years of age" refers to an experimental trial having been carried out in human subjects that indicated that the pharmaceutical composition is effective in treating persons in the recited age range.

As used herein, "cervical" refers to the uterine cervix.

Determining the type or types of HPV present in an individual refers to carrying out a test to identify an individual as having and/or not having a nucleic acid sequence and/or an amino cid sequence from a particular type or types of HPV. Typing of HPV in a person can be carried out, for example, by PCR. The typing procedure does not necessarily identify an HPV type of an active HPV infection, but includes the identification of HPV nucleic acid sequence and/or amino acid sequence present in an individual as a result of an earlier infection that no longer produces active viral particles.

An "HPV-mediated disease" is any disease or medical condition caused by and/or associated with an infection of a human papillomavirus. Examples of HPV-mediated diseases include, but are not limited to, HPV-associated infections, atypical squamous cells of undetermined significance (ASCUS), warts (e.g., anogenital warts, bowenoid papulosis, giant condylomata, cutaneous warts, common warts, plantar warts, flat warts, butcher warts, and epidermodysplasia verruciformis), respiratory papillomatosis, laryngeal papilloma, maxiallary sinus papilloma, conjunctival papillomatosis, oral focal hyperplasia, intraepithelial neoplasia (e.g., cervical intraepithelial neoplasia (CIN), vulval intraepithelial neoplasia (VIN), and anal intraepithelial neoplasia (AIN)), cervical cancer, vulvar cancer, anal cancer, vaginal cancer, penile cancer, head and neck cancer, squamous cell carcinoma of the lung, squamous cell carcinoma of the sinuses, squamous cell carcinoma of the esophagus, oral carcinoma, conjunctival carcinoma, and other HPV-mediated cancers. The compositions applied in the uses of the present invention are for treating CIN.

The compositions described herein can be used to treat or prevent CIN associated with HPV types 16 and 18, as well as other HPV types including but not limited to HPV types 6, 11, 31, 33, 35, 39, 44, 45, 52, 53, 54, 56, 58, 61, 66, 67, 69. CP8304, CP141, MM4, MM7, _ and/or MM9. Conditions associated with specific HPV types include high grade cervical intraepithelial neoplasia (HPV 31, 33, 35, 39, 44, 45, 52, 53, 54, 56, 58, 61, 88. 67, 69, CP8304, CP141, MM4, MM7, and MM9).

In some embodiments of any of the uses or compositions applied in the uses described herein, the CIN can be any of cervical intraepithelial neoplasia 1 (CIN1) or low grade squamous intraepithelial lesion (LSIL). In other embodiments of any of the uses or compositions applied in the uses, the CIN can be any of cervical Intraepithelial neoplasia 2 (CIN2), cervical lntraepithelial neoplasia 3 (CIN3), cervical intraepithelial neoplasia 2/3 (CIN2/3), or high grade sqamous intraepithelial lesion (HSIL).

In the uses of the present invention, an individual is identified as being less than 25 years of age or as being 18 to 24 years of age. Alternatively, an individual is identified as being 12-20, 16-20 or 18-20 years of age.

The uses described herein include, after the administering step, observing an elimination or a reduction in the severity of the CIN, a symptom of the CIN, or both.

In the uses or compositions applied in the uses described herein, the epitope is a peptide that binds to the binding groove of an MHC class I molecule.

In the uses or compositions applied in the uses described herein; the epitope is a peptide that binds to the binding groove of an MHC class II molecule.

In the uses or compositions applied in the uses described herein, a hybrid polypeptide comprises an epitope from an HPV strain 16 E6 protein, an epitope from an HPV strain 16 E7 protein, an epitope from an HPV strain 18 E6 protein, and an epitope from an HPV strain 18 E7 protein. For example, a hybrid polypeptide can comprise a segment of an HPV strain 16 E6 protein that is at least eleven amino acids in length and comprises two epitopes, a segment of an HPV strain 16 E7 protein that is at least eleven amino acids in length and comprises two epitopes, a segment of an HPV strain 18 E6 protein that is at least eleven amino acids in length and comprises two epitopes, and a segment of an HPV strain 18 E7 protein that is at least eleven amino acids in length and comprises two epitopes.

As used herein, a "segment" is an amino acid sequence which (a) corresponds to the sequence of a portion (i.e., fragment less than all) of a naturally occurring HPV protein, and (b) contains one or more epitopes. The term "segment" is used herein to denote a part of the hybrid polypeptide, while the term "portion" is used to denote the corresponding part of the naturally occurring HPV protein. A methionine codon is included at the 5' end of a sequence of a nucleic acid to facilitate translation. In addition, the hybrid polypeptide can encode a targeting signal, as described in more detail below. Adjacent segments can be contiguous, or can be separated by a spacer amino acid or spacer peptide, as defined below.

A hybrid polypeptide may optionally include additional segments, e.g., it can include at least 4, 5, 10, 15, 20, 25, 30, 40, 50, 60, 75, 90, or even 100 or more segments, each being a portion of a naturally-occurring protein of a naturally occurring HPV protein which can be the same or different from the protein(s) from which the other segments are derived. Each of these segments can be at least 8, 9, 10, 11, 12, 13, 14, 15, or more amino acids in length, and each contains at least one epitope (preferably two or more) different from the epitopes of the first, second, and third segments. At least one (preferably at least two or three) of the segments in the hybrid polypeptide may contain, e.g., 3, 4, 5, 6, 7, or even 10 or more epitopes, e.g., class I or class II MHC-binding epitopes. Two, three, or more of the segments can be contiguous in the hybrid polypeptide (i.e., joined end-to-end, with no spacer between them). Alternatively, any two adjacent segments can be linked by a spacer amino acid or spacer peptide.

A given segment within the hybrid polypeptide need not be any specified length, so long as it is sufficiently long to generate at least one epitope, e.g., 2, 3, 4, 5, or more epitopes. A given segment can have a length of at least 8 amino acids, e.g., at least 11, 12, 13, 14, 15, 20, 25, 30, 40, or 50 amino acids. A given segment corresponds to a particular naturally occurring HPV protein if any number of consecutive amino acids of the segment are found in exactly the same order in a portion of the naturally occurring protein. The lengths of HPV strain 16 E6 and E7 proteins are 158 and 98 amino acids, respectively, and the lengths of the HPV strain 18 E6 and E7 proteins are 158 and 105 amino acids, respectively. The segment is optionally less than 100 amino acids (less than 95 amino acids for the HPV strain 16 E7 protein), less than 70 amino acids, or less than 50 amino acids (e.g., 20-50). In one embodiment, it is less than 15. The segments within the hybrid polypeptide can be arranged in any order within the polypeptide. The hybrid polypeptide optionally does not contain a sequence identical to the sequence of either full length, intact E6 or full length, intact E7 protein from HPV strain 16 or 18.

A hybrid polypeptide can contain a first epitope from an HPV protein and a second epitope that does not overlap with the first epitope and is from the same or a different HPV protein ("different HPV protein" can include a non-identical homolog of the first protein, derived from a different HPV strain than that from which the first protein was derived.) The first epitope can bind to a first major histocompatibility complex (MHC) class I allotype and the second epitope binds to a second MHC class I allotype different from the first MHC class I allotype.

One or more of the epitopes of the hybrid polypeptide can be from the HPV E6 or E7 protein, and the protein can be from HPV strain 16 or 18. Thus, the hybrid polypeptide can include, e.g., one or more segments from each of the HPV strain 16 E6 and E7 proteins, and one or more segments from each of the HPV strain 18 E6 and E7 proteins. The hybrid polypeptide can be at least 15 amino acids in length, and can be, e.g., at least 20, 25, 33, 34, 35, 40, 44, 45, 50, 75, 100, 150, 200, 250, 300, 400, 500 or 750 amino acids.

The MHC class I allotypes to which the epitopes in the hybrid polypeptide bind can be any human class I allotypes, e.g., HLA-A1, HLA-A2, HLA-A3, HLA-A11. and/or HLA-A24. A given epitope may be promiscuous, i.e., bind more than one allotype.

The hybrid polypeptide may also include a third epitope of an HPV protein. This epitope, which can be derived from the same or a different HPV protein as the first and/or second epitope, binds to a third MHC class I allotype different from the first and second MHC class I allotypes. It may, of course, also bind to the first and/or second MHC class I allotype, in addition to the third. The hybrid polypeptide may include, e.g., at least 4, 5, 6, 7, 8, 9, 10, 15, 25, 35, 40, 50, 60, 80, or even 100 or more MHC class I allotype-binding epitopes from one or more HPV proteins. Some of these epitopes may overlap.

The polypeptide may optionally include a targeting signal. A targeting signal is a peptide which directs intracellular transport or secretion of a peptide to which it is attached. The targeting signal can be at the amino terminus, e.g., a signal sequence, or carboxy terminus, or within the hybrid polypeptide, so long as it functions in that site. A preferred targeting signal is the signal peptide of HLA-DRα: Met Ala Ile Ser Gly Val Pro Val Leu Gly Phe Phe Ile Ile Ala Val Leu Met Ser Ala Gin Glu Ser Trp Ala. The targeting signal may optionally be modified to introduce an amino acid substitution at the junction(s) between the targeting signal and the adjacent segment(s) to promote cleavage of the targeting sequence from the epitopes by, e.g., a signal peptidase.

Any of the segments within the hybrid polypeptide may be separated from the others by a spacer amino acid or a spacer sequence. By "spacer amino acid" is meant a single residue inserted between two neighboring segments ("A" and "B", in that order) in a polypeptide, where the residue is different from the amino acid which flanks the carboxy terminus of A and also is different from the amino acid which flanks the amino terminus of B in the respective full length proteins from which A and B were derived ("X" and "Y", respectively). By "spacer sequence" is meant a sequence of two or more amino acid inserted between two neighboring segments. Spacer amino acids and spacer sequences are useful for promoting processing to release epitopes. The spacers are typically removed from the polypeptide by proteolytic processing in the cell, along with any sequence between epitopes within a given segment. This leaves the epitopes intact for binding to MHC molecules or (upon secretion from the cell) antibodies. Occasionally a spacer amino acid or part of a spacer sequence will remain attached to an epitope through incomplete processing. This generally will have little or no effect on binding to the MHC molecule.

The nucleic acid may encode a hybrid polypeptide including a first epitope and a second epitope, wherein the first epitope is from a first HPV protein and the second epitope is from a second HPV protein different from the first HPV protein. The sequence of the second epitope can optionally not occur within the first HPV protein, i.e., they can be derived from different proteins. The different proteins can be from the same or different strains of HPV, e.g., types 16 and 18. The hybrid polypeptide can, of course, include additional epitopes from the same or different HPV proteins, or from other pathogens or tumor antigens.

The nucleic acid can also encode a hybrid polypeptide including a plurality (e.g., at least 2, 3, 4, 5, 6, 7, 8, 9, 10, 15, 20, or 30) MHC class I-binding epitopes from an HPV protein. The sequence of the entire hybrid polypeptide is optionally not identical to the sequence of either a naturally occurring HPV protein or a fragment of
a naturally occurring HPV protein, by virtue of sequence insertions, internal deletions, or substitutions, accomplished, e.g., by genetic engineering techniques.

The nucleic acid may encode a hybrid polypeptide that includes a plurality of HLA-binding epitopes from an HPV strain 16 E6 protein, a plurality of HLA-binding epitopes from an HPV strain 16 E7 protein, a plurality of HLA-binding epitopes from an HPV strain 18 E6 protein, and a plurality of epitopes from an HPV strain 18 E7 protein. Each plurality of epitopes can include an epitope selected from the group consisting of an HLA-A1-binding epitope, an HLA-A2-binding epitope, an HLA-A3-binding epitope, an HLA-A11-binding epitope, and an HLA-A24-binding epitope, and preferably at least two or three selected from this group. The members of each plurality of epitopes are different from the members of each of the other plurality of epitopes. Each plurality of epitopes may include at least two HLA-A1-binding epitopes, at least two HLA-A2-binding epitopes, at least two HLA-A3-binding epitopes, at least two HLA-A11-binding epitopes, and/or at least two HLA-A24-binding epitopes. A given plurality can be distributed on more than one segment.

The nucleic acid encoding a hybrid polypeptide can optionally contain the segments AMFQDPQERPRKLPQLCTEL, LLRREVYDFAFRDLCIVYRDGNPY, KISEYRHYCYSLYGTTLEQQYNK, TLHEYMLDLQPETTDLYSY, QAEPDRAHYNIVTF, LLMGTLGIVCPICSQKP, RRPYKLPDLCTELNTSLQDIEITCVYCKTVLELTEVFEFAFK, SVYGDTLEKLTNTGLYNLLIRCLRCQK, KATLQDIVLHLEPQNEIPV, HTMLCMCCKCEARI, and AFQQLFLNTLSFVCPWC. The segments can be processed to produce multiple epitopes. The hybrid polypeptide can include one or more of the peptide segments, as well as additional HPV E6 or E7 sequence. The hybrid polypeptide optionally does not contain a sequence identical to the sequence of either full length, intact E6 or full length, intact E7 protein from HPV strain 16 or 18.

The nucleic acid can optionally be an "isolated" nucleic acid. As used herein, "isolated" nucleic acid covers both (1) a nucleic acid the full sequence of which does not occur within any naturally occurring nucleic acid, and (2) a nucleic acid the full sequence of which does occur within a naturally occurring nucleic acid, but which is free of the genes that flank that sequence in the genome of the organism in which that sequence naturally occurs. The term therefore includes a recombinant nucleic acid incorporated into a vector, into an autonomously replicating plasmid or virus, or into the genomic DNA of a prokaryote or eukaryote. It also includes a separate molecule such as a cDNA, a genomic fragment, a fragment produced by polymerase chain reaction (PCR), or a restriction fragment.

The term "nucleic acid" as used herein includes, for example, ribonucleotides, deoxyribonucleotides, or modified forms of either nucleotide. Isolated nucleic acid sequences can be single or double stranded and can be polynucleotides or oligonucleotides. Isolated nucleic acids as used herein may be provided as a plasmid, bacterial, or viral vector. The vectors may be expression vectors that permit expression in a cell of interest.

In the uses or compositions applied in the uses described herein, the polypeptide is a hybrid polypeptide that further comprises a signal sequence.

In the uses or compositions applied in the uses described herein, the polypeptide is a hybrid polypeptide comprising at least one of the following segments of HPV strain 16 E6:
AMFQDPQERPRKLPQLCTEL,
LLRREVYDFAFRDLCIVYRDGNPY, or
KISEYRHYCYSLYGTTLEQQYNK;
at least one of the following segments of HPV strain 16 E7:
TLHEYMLDLQPETTDLYSY.
OAEPDRAHYNIVTF, or
LLMGTLGIVCPICSQKP;
at least one of the following segments of HPV strain 18 E6:
RRPYKLPDLCTELNTSLQDIEITCVYCKTVLELTEVFEFAFK, or
SVYGDTLEKLTNTGLYNLLIRCLRCQK,
and at least one of the following segments of HPV strain 18 E7:
KATLQDIVLHLEPQNEIPV,
HTMLCMCCKCEARI, or
AFQQLFLNTSFVCPWC.

In some of the uses or compositions applied in the uses described herein, the polypeptide: (1) comprises the amino add sequence
AMFQDPQERPRKLPQLCTELLLRREVYDFAFRDLCIVYRDGNPYKISEYRHYCYSLYGTTLE QQYNKTLHEYMLDLQPETTDLYSYQAEPDRAHYNIVTFLLMGTLGIVCPICSQKPRRPYKLPD LCTELNTSLQDIEITCVYCKTVLELTEVFEFAFKSVYGDTLEKLTNTGLYNLLIRCLRCQKKATL QDIVLHLEPONEIPVHTMLCMCCKCEARIAFQQLFLNTLSFVCPWC; (2) comprises the amino acid
sequence MAISGVPVLGFFIIAVLMSAQESWAAMFQDPQERPRKLPQLCTELLLRREVYDFAF RDLCIVYRDGNPYKISEYRHYCYSLYGTTLEQQYNKTLHEYMLDLQPETTDLYSYQAEPDRA HYNIVTFLLMGTLGIVCPICSQKPRRPYKLPDLCT ELNTSLQDIEITCVYCKTVLELTEVFEFAFKSVYGDTLEKLTNTGLYNLLIRCLRCQKKATLQDI VLHLEPQNEIPVHTMLCMCCKCEARIAFQQLFLNTLSFVCPWC; or (3) consists of the amino acid sequence
MAISGVPVLGFFIIAVLMSAQESWAAMFQDPQERPRKLPQLCTELLLRREVYDFAFRDLCIVY RDGNPYKISEYRHYCYSLYGTTLEQQYNKTLHEYMLDLQPETTDLYSYQAEPDRAHYNIVTF LLMGTLGIVCPICSQKPRRPYKLPDLCTELNTSLQDIEITCVYCKTVLELTEVFEFAFKSVYGDT LEKLTNTGLYNLLIRCLRCQKKATLQDIVLHLEPQNEIPVHTMLCMCCKCEARIAFQQLFLNTL SFVCPWC.

In some of the uses or compositions applied in the uses described herein, the polypeptide encoded by the nucleic acid does not comprise a heat shock protein or an immunostimulatory fragment of a heat shock protein.

In some of the uses or compositions applied in the uses described herein, the polypeptide encoded by the nucleic acid does not comprise an amino acid sequence that functions as an adjuvant.

In some of the uses or compositions applied in the uses described herein, the polypeptide encoded by the nucleic acid consists of any of the amino acid sequences described herein.

In some of the uses or compositions applied in the uses described herein, the nucleic acid comprises an expression vector, e.g., a plasmid vector or a viral vector (e.g., a vaccinia virus, a lentivirus, an alphavirus, a pox-virus, a retrovirus, an adenovirus, or an adeno-assodated virus).

In some of the uses and compositions applied in the uses described herein, the pharmaceutical composition does not comprises a virus, e.g., a vaccinia virus or a pox virus.

In the uses or compositions applied in the uses described herein, the pharmaceutical composition may comprise a microparticle. Accordingly, for example the pharmaceutical composition may comprise a microparticle having the nucleic acid encapsulated therein, e.g., having the nucleic acid (e.g., viral, bacterial, or plasmid vectors) encapsulated in a polymeric matrix.

A polymeric matrix of a microparticle can comprise, consist of, or consist essentially of poly-lactic add (PLA), poly-glycolic acid (PGA), or a copolymer of poly-lactide-co-glycolide acid (PLGA). The microparticle can have a diameter of, e.g., 0.02 to 20 microns, or less than about 11 microns. A plurality of the microparticles optionally has an average diameter of, e.g., 0.02 to 20 microns, less than about 11 microns, or less than about 5 microns.

The nucleic acids described herein can alternatively be incorporated into liposomes or immune-stimulating complexes (ISCOMS) or delivered with naturally occurring polymers, synthetic polymers, biopolymers, cationic lipids, condensing agents, dendrimers, other biomaterials, oil-containing adjuvants, and other adjuvants such as QS21 or saponin. The nucleic acids described herein may alternatively be administered using any other suitable delivery vehicle known in the art, or can be delivered without a delivery vehicle (other than aqueous solution), e.g., naked DNA".

In some of the uses or compositions applied in the uses described herein, the pharmaceutical composition comprises an adjuvant, e.g., an immune modulator such as GMCSF, imiquimod, or resiquimod.

In some of the uses or compositions applied in the uses described herein, the pharmaceutical composition is co-administered together with an adjuvant. In such a co-administration, the adjuvant need not be administered at the same time as the pharmaceutical composition. For example, the adjuvant can be co-administered within, for example, 1, 2, 3, 6, or 12 hours, or 1, 2, 3, 4, 5, 6, or 7 days (before or after) of the administration of the pharmaceutical composition.

In some of the uses described herein, the administration is performed via injection, e.g., intramuscular injection, subcutaneous, intracervical, or a combination thereof.

An advantage of the invention is that it permits the treatment of an individual having CIN, without the need to first identify the type or types of HPV that have infected the individual. As detailed herein, it has been unexpectedly found that a pharmaceutical composition containing a nucleic acid encoding a polypeptide containing epitopes from two specific types of HPV is effective in treating CIN caused by HPV types other than the two HPV types represented in the encoded polypeptide. Such an unexpected cross-reactive immune response allows for the use of a pharmaceutical composition that can be broadly effective against infection by a variety of HPV types.

Another advantage of the invention is that it allows for the selection of a specific category of individuals that are particularly amenable to successful treatment with a pharmaceutical composition containing a nucleic acid encoding a polypeptide containing an epitope of an HPV protein. As detailed herein, it has been unexpectedly found that such a pharmaceutical composition is effective in treating CIN in persons being less than 25 years. As a result of this unexpected discovery, individuals that are expected to specifically benefit from the treatment can be selected based upon their age, and those that are not expected to benefit (based upon age criteria) can be excluded from the treatment program and can be assigned to other available therapies that are appropriate for their specific age group.

Unless otherwise defined, all technical and scientific terms used herein have the same meaning as commonly understood by one of ordinary skill in the art to which this invention belongs. Suitable methods and materials are described below, although methods and materials similar or equivalent to those described herein can also be used in the practice or testing of the present invention. In case of conflict, the present specification, including
definitions, will control. In addition, the materials, methods, and examples are illustrative only and not intended to be limiting.

Other features and advantages of the invention will be apparent from the following detailed description, and from the claims.

### Brief Description of the Drawings

Fig. 1 is a chart depicting a schedule of study events.
Fig. 2 is a panel of graphs depicting T cell responses to HPV type 16, HPV type 18, HPV type 6, and HPV type 11 peptides following treatment of individuals with ZYC101a.
Fig. 3 is a graph depicting the change in tumor size *in vivo* over time following treatment with ZYC101a or ZYC101a combined with electroporation.

### Detailed Description

### Identification of MHC-Binding Epitopes from HPV Proteins

For an epitope to generate an effective T cell response, it binds to an MHC molecule on an antigen-presenting cell (APC), such that the resulting receptor-ligand complex (MHC-peptide complex) is then recognized by a T cell receptor expressed on a T cell (e.g., a cytotoxic T cell; CTL).

Peptide epitopes that bind to specific MHC alloforms can be identified by extraction of peptides from MHC class I and/or class II molecules, followed by mass spectrometry analysis to determine the amino acid sequence of the the bound peptide or peptides. Such methods are described in detail in, e.g., U.S. Patent No. 5,827,516 and WO 00/09654.

In addition, epitopes can be identified by synthesizing a series of peptide fragments (e.g., overlapping peptide fragments) from an HPV protein of interest, e.g., HPV E6 or E7, and testing the peptides in binding studies with a radiolabeled peptide known to bind to an MHC alloform. If a peptide demonstrates specific binding to an MHC alloform (i.e., it is determined to be an epitope), e.g., as measured by competition with the radiolabeled peptide, then the peptide epitope can optionally be combined with one or more additional epitopes (overlapping or adjacent) to produce a segment contained in a hybrid polypeptide. Alternatively, a nucleic acid used in the methods described herein can encode a polypeptide containing only a single HPV epitope.

MHC class I epitopes can be identified by refolding soluble MHC molecules in the presence of radiolabeled β2-microglobulin and a test peptide. An MHC-peptide-β2-microglobulin complex refolds and produces a protein complex of a predicted size. β2-microglobulin dissociates from the complex at a measurable rate that is directly correlated with the binding affinity of the test peptide (Garboczi et al., Proc. Nat. Acad. Sci. USA 89:3429-33, 1992; Parker et al., J. Biol. Chem. 267:5451-5459, 1992; and Parker et al., J. Immunol. 149: 1896-1904, 1992).

Binding studies on putative HPV epitopes, e.g., HPV 18 E6- and/or E7- derived epitopes, can be performed using, for example, overlapping 8, 9, 10, 11, 12, 13, 14, or 15-mers from the HPV 18 E6 and/or E7 proteins in binding assays using one or more HLA molecules.

A variety HPV peptide epitopes have been characterized using methods such as those described herein. Examples of specific HPV epitopes (e.g., HPV strain 16 and 18 E6 and E7 derived epitopes) are described in, e.g., U.S. Patent No. 6,183,746, WO 01/19408, U.S. Patent No. 6,037,135, WO 99/45954, WO 99/22338, and WO 97/33602. Non-limiting examples of MHC class I-binding HPV peptides, which can be included in a polypeptide encoded by a nucleic acid described herein, include:
(1) ISEYRHYCY, FQDPQERPR, RREVYDFAF, TTLEQQYNK, FQDPQERPRK, ISEYRHYCYS, KISEYRHYCY, GTTLEQQYNK, KLPQLCTEL, KISEYRHYC, FAFRDLCIV, YCYSIYGTTL, SEYRHYCYSL, AMFQDPQER, LLRREVYDF, IVYRDGNPY, VYDFAFRDL, CYSLYGTTL, EYRHYCYSL, KLPQLCTEL, DPQERPRKL, HYCYSLYGT, DFAFRDLCI, LYGTTLEQQY,
   HYCYSLYGTT, EVYDFAFRDL, EYRHYCYSLY, VYDFAFRDLC (HPV 16 E6 epitopes);
(2) OAEPDRAHY, IVCPICSQK, QPETTDLY, QAEPDRAHYN, DLQPETTDLY, YMLDLQPET, TLHEYMLDL, LLMGTLGIV, LMGTLGIVC, MLDLQPETT, TLGIVCPIC, DLQPETTDL, GTLGIVCPI, YMLDLQPETT, LQPETTDLY, LLMGTLGIVC, DLQPETTDL, TLHEYMLDL, TPTLHEYML, RAHYNIVTF, and EPDRAHYNI (HPV 16 E7 epitopes);
(3) LTEVFEFAFK, KLPDLCTEL, GLYNLLIRC, SLQDIEITC, SLQDIEITCV, LQDIEITCV, KTVLELTEV, ELTEVFEFA, KLTNTGLYNL, LTNTGLYNL, GLYNLLIRCL, VLELTEVFEF, SVYGDTLEK , LLIRCLRCQK, VYCKTVLEL, VYGDTLEKL, and LTNTGLYNLL (HPV 18 E6 epitopes); and
(4) HLEPQNEIPV, TLQDIVLHL, ATLQDIVLHL, QLFLNTLSFV, MLCMCCKCEA, CMCCKCEARI, FQQLFLNTL, TLSFVCPWC, HTMLCMCCK, QLFLNTLSF, and AFQQLFLNTL (HPV 18 E7 epitopes).

The epitope characterization methodologies described herein can be used to determine whether a nucleic acid encoding a polypeptide containing one or more HPV epitopes will generate a cross-reactive T cell response that recognizes a target cell expressing a polypeptide from another HPV type. *In vitro* T cell assays as well as *in vivo* studies can also be used to assess such cross reactivity.

Peptide epitopes that are isolated from MHC molecules, bind *in vitro* to MHC molecules, and/or are predicted to bind to MHC molecules can be analyzed for their effectiveness at stimulating a T cell-response in an *in vitro* immunization assay (IVI). Effectiveness in an IVI assay can optionally be used as a criterion for inclusion of a sequence encoding a peptide in a nucleic acid construct described herein. IVI assays have been used to identify human and murine T cell-responsive epitopes, including several that are derived from HPV proteins (Alexander et al., Amer. J. Obstet. and Gynecol. 175:1586-1593, 1996; Tarpey et al., Immunology 81:222-27, 1994). The first round of T cell stimulation in an IVI assay can optionally be performed in the presence of dendritic cells (DCs) pulsed with a test peptide. In addition, inclusion of IL-10 during the stimulation may suppress non-specific responses that may sometimes arise during culture of the cells. T cell activation can then be examined by ELISA (to measure γ-IFN secretion), FACS (to determine the increase in CD8+, CD16- cells containing γ-IFN by tricolor analysis), ⁵¹Cr release CTL assay, ELISpot, or a tetramer-based assay (see, e.g., Molldrem et. al. (2000) Nature Med. 6:1018).

A ⁵¹Cr release CTL assay can be performed by incubating cells with a vaccinia virus (encoding a polypeptide containing at least one epitope of an HPV protein) for 2.5 hours in the presence of ⁵¹Cr. Following washing, the vaccinia-infected cells are contacted for 5 hours with T cells (e.g., autologous T cells) previously activated with peptide. ⁵¹Cr release into supernatant is taken as a measure of target cell lysis by the effector cells. Target cells infected with a vaccinia vector encoding the test peptide are more efficiently lysed by the effector cells than are control target cells (infected with an empty vaccinia vector), indicating that the peptide is expressed within the cells and effectively presented to T cells by the cells' HLA molecules.

ELISpot (enzyme-linked immunospot assay) measures secretion of a test cytokine by effector T cells in response to a specific antigen. T cell populations are incubated *in vitro* with an equal number of syngeneic stimulator cells that have been pre-pulsed with a T cell peptide epitope or infected with a recombinant vaccinia virus that expresses the peptide. After 24 hours of co-culture, T cell activity is determined by performing the ELISpot assay according to the manufacturer's instructions (Human or Mouse IFN-gamma ELISpot, R&D Systems). The spots, representing the frequency of reactive T-cells are counted in a commercially available computer-automated ELISpot reader system.

Where it is desirable to induce a cross-reactive T cell response, the cross-reactivity can be tested in an IVI assay. For example, an HPV16 or HPV18 peptide can be used as the stimulating peptide to produce activated T cells. In the testing phase (e.g., ELISpot), the T cells activated by the HPV16 or HPV18 peptide are tested for their ability to respond to a peptide from a different HPV type (e.g., HPV type 6, 11, 31, 33, 35, 39, 44, 45, 52, 53, 54, 56, 58, 61, 66, 67, 69, CP8304, CP141, MM4, MM7, or MM945). A response to the non HPV16/18 peptide demonstrates a cross-reactive T cell response to cells expressing non-HPV type 16 or 18 HPV proteins. Such analyses can be useful in determining whether a particular nucleic acid (e.g., a nucleic acid encoding a polypeptide containing particular segments of the HPV16 and HPV18 E6 and E7 proteins) will generate a T cell response that cross-reacts with other HPV types. Such cross reactive T cell responses can allow a single nucleic acid to be used to treat HPV-mediated diseases caused by different types of HPV. T cells from multiple donors (e.g., two, three, four, or more donors) for each HLA allotype (e.g., HLA-A2 and HLA-A3) can optionally be tested in an IVI assay to confirm broad T cell reactivity to a given peptide epitope combined with a particular HLA allotype.

### Nucleic Acids Encoding Polypeptides Containing One or More Epitopes from an HPV Protein

A nucleic acid encoding a polypeptide containing HPV epitopes can be generated using standard techniques, e.g., by overlapping PCR or linking of nucleotide sequences such as oligonucleotides. Preferably, codons are selected for inclusion in the construct to optimize production of the polypeptide in mammalian systems. In some embodiments, the nucleic acid encodes a polyepitope polypeptide, which contains a plurality of HPV epitopes.

In those embodiments where the nucleic acid encodes a polyepitope polypeptide, different segments in the encoded polyepitope polypeptide, e.g., segments derived from different HPV proteins, or from non-adjacent regions of the same HPV protein, can be checked using a sequence analysis program, e.g., the BLAST program, to determine if the amino acid sequences at the junctions of the segments show inadvertent similarity to known human proteins. If homologies exist, the polypeptide can be redesigned to alter the order of the epitopes in the polypeptide to eliminate the region of homology.

Epitope-containing segments derived from a single HPV protein can appear in the order (i.e., from amino terminus to carboxy terminus) in which they appear in the naturally occurring HPV protein. Alternatively, segments from a given HPV protein can be arranged in an order other than that in which they appear in the native HPV protein, and can be grouped together or mixed with segments from one or more other HPV proteins. If desired, a spacer amino acid or spacer sequence (e.g., alanine spacers) can be inserted between each pair of segments (Toes et al., Proc. Nat. Acad. Sci. (USA) 94:14660-65, 1997).

In some examples, a nucleic acid can encode a single polypeptide or multiple polypeptides (e.g., multiple polyepitope polypeptides), each under the control of a different promoter, e.g., a dual promoter vector. For example, a dual promoter vector can permit two shorter polyepitope polypeptides to replace a single longer version, with no loss in the number of epitopes produced from a given vector.

For nucleic acids encoding a polypeptide containing an epitope from an HPV E7 protein, the nucleic acid optionally does not encode a peptide corresponding to the retinoblastoma protein (Rb)-binding site in the HPV E7 protein.

A nucleic acid can encode a polypeptide containing 3, 4, 5, 6, 7, 8, 9,10, 15, 20, 25, 30, 35, 40, or more epitopes derived from 4, 5, 6, 7, 8 or more naturally occurring HPV proteins. Exemplary HPV epitopes that can be encoded by a nucleic acid, as well as exemplary portions of HPV proteins that contain a plurality of epitopes, are described herein. A nucleic acid of the present invention encodes a polypeptide containing epitopes (e.g., CTL epitopes) derived from an E6 and E7 HPV protein of HPV types 16 and 18. In addition, it can encode a polypeptide containing epitopes (e.g., CTL epitopes) derived from HPV proteins of any HPV type, including but not limited to HPV types 6, 11, 16, 18, 31, 33, 35, 39, 44, 45, 52, 53, 54, 56, 58, 61, 66, 67, 69, CP8304, CP141, MM4, MM7, and MM945. HPV proteins that can be used to derive the peptide epitopes include, for example, E6, E7, E1, E2, E4, E5, L2, and L1. For example, a polyepitope polypeptide can include at least 4 segments from each of the HPV 16 E6, HPV 16 E7, HPV 18 E6, and HPV 18 E7 proteins, where each segment contains epitopes (e.g., at least 2, 3, 4, 5, 6, 7, 8, 9, 10, or more epitopes) for binding to one or more of the HLA alleles HLA-A 1, HLA-A2, HLA-A3, HLA-A11, and/or HLA-A24.

Hybrid polyepitope polypeptides can include multiple immunogenic segments from different HPV proteins, each segment containing one or more MHC class I and/or II-restricted epitope. A hybrid polypeptide can include segments from the E6 and E7 proteins of HPV16 and HPV18. One or more of the segments present within the polyepitope polypeptide can be processed into multiple distinct epitopes to form MHC class I-binding epitopes, which are typically associated with T cells that secrete gamma interferon and can have cytotoxic capabilities (e.g., a CTL), and/or MHC class II-binding epitopes, which are typically associated with helper T cell immune responses such as the secretion of cytokines that mediate the immune response and affect the local or systemic environment. Other epitopes can be B cell epitopes that stimulate production of epitope-specific antibodies. Nucleic acids encoding polyepitope polypeptides described in WO 01/19408, are particularly useful in the methods of the invention.

In those instances where it is desirable to elicit a cross-reactive T cell response, a nucleic acid can be constructed, for example, (1) to include segments of HPV16 and HPV18 E6 and E7 proteins that contain a plurality of epitopes that induce a cross-reactive T cell response as determined in an IVI assay, and/or (2) ordering segments in the nucleic acid in a manner that creates the greatest degree of cross-reactivity when tested empirically in T cell assays (e.g., such that T cells generated in mice transgenic for HIA-A2, following administration of the nucleic acid, are able to recognize a broad array of HLA-A2 binding peptides from HPV types other than HPV types 16 and 18).

Regulatory elements can be included in the construct to facilitate expression of the nucleic acid encoding the polypeptide. These elements include sequences for enhancing expression in human or other mammalian cells, e.g., promoters, RNA stabilization sequences 5' and/or 3' to the coding sequence, introns (which can be placed at any location within or adjacent to the encoded sequence), and poly(A) addition sites, as well as an origin of replication and one or more genes encoding
selectable markers enabling the constructs to replicate and be selected in prokaryotic and/or eukaryotic hosts. A T7 polymerase promoter or other type of promoter (e.g., a tissue-specific promoter such as a muscle-specfic promoter, or a cell-specfic promoter such as an APC-specfic promoter) is optionally present at the 5' end of the coding sequence, and a sequence encoding a FLAG or other antibody determinant is optionally present directly 3' of ' the last epitope coding sequence. The construct may also contain other transcriptional and translational signals, such as a Kozak sequence.

The construct may in addition include a sequence encoding a targeting signal that directs the polypeptide to a desired intracellular compartment, the targeting signal being linked to the polypeptide. Targeting signals can direct the polypeptide to the endoplasmic reticulum (ER), golgi, nucleus, a lysosome, a class 11 peptide loading compartment, or an endosome, and include signal peptides (the amino terminal sequences which direct proteins into the ER during translation; also known as a signal sequence). Also included are targeting signals that direct insertion of the polypeptide into a membrane (e.g., a transmembrane sequence). Polypeptides including a membrane insertion sequence can be constructed either with or without a cytoplasmic tail.

An example of an ER-targeting sequence is the HLA-DRa leader sequence (MAISGVPVLGFFIIAVLMSAQESWA). The targeting sequence may include only a portion (e.g., at least ten amino acid residues) of this specified 25 residue sequence, or a slightly modified version of this sequence, provided that the portion or modified sequence is sufficient to cause targeting of the polypeptide to the ER.

Nuclear localization sequences include nucleoplasmin- and SV40-like nuclear targeting signals, as described in Chelsky et al., Mol. Cell Biol. 9:2487, 1989; Robbins, Cell 64:615, 1991, and Dingwall et al., TIBS 16:478, 1991.

In some cases it is desirable to modify the amino acid sequence of the targeting signal to facilitate cleavage by a signal peptidase or other proteolytic agent. Recognition sequences for signal peptidases are described in Von Heijne, Nucleic Acids Research 14:4683, 1986. The -3, -1 rules of von Heijne can be used to select a sequence that increases the probability of successful cleavage by signal peptidase when the targeting signal is present.

A nucleic acid encoding a polypeptide containing HPV epitopes also encodes a methionine residue at the amino terminus of the polypeptide to facilitate translation.

A nucleic acid encoding a polypeptide containing HPV epitopes can be used in any vector that allows for expression in an antigen-presenting cell (APC) of a an individual. Useful vectors include a vaccinia virus, a lentivirus, an alphavirus (e.g., Sindbis virus (SV40), the Semliki Forest virus, the Ross River virus, and the Venezuelan, Western and Eastern equine encephalitis viruses), a pox-virus, a retrovirus (e.g., a lentivirus such as HIV-1, HIV-2, or SIV), an adenovirus, and an adeno-associated virus (AAV) based vector (see, e.g., West et al., Virology 160:38-47 (1987), U.S. Patent No. 5,173,414, Panicali et al., J. Virol. 37:1000-1010 (1981). U.S. Patent No. 6,013,576, WO 96/25507, and WO 92/10578).

The vector can be a non-integrating viral vector or a non-viral vector such as a plasmid or bacterial vector. Examples of a suitable plasmid vectors include the family of pcDNA mammalian expression vectors (Invitrogen), which permit direct and rapid cloning of PCR products.

To determine whether an encoded polypeptide is processed in a cell and the expected epitopes are presented by HLA, an *in vitro* T cell stimulation assay can be performed using autologous PBL or EBV-transformed cells infected with a recombinant vaccinia virus that contains the polypeptide coding sequence. The target cells can be generated by incubating PBL with recombinant vaccinia at an m.o.i of 3-10 pfu/cell at 37°C for 2 hours. After infection, cells are pelleted, washed and used as targets in an *in vitro* stimulation assay. The stimulated T cells from one or more individuals (e.g., with the different HLA allotypes) are incubated with the target cells, and the ability of the target cells to stimulate the T cells is measured, e.g., by γ-interferon expression or secretion.

Alternatively, epitope processing from the polypeptide can be examined using proteasomes purified from human cells (Theobald et al., J. Exp. Med. 188:1017, 1998;
Kisselev et al., J. Biol. Chem. 289:3363, 1999; and Nussbaum et al., Proc. Nat. Acad. Sci. (USA) 95:12404, 1998).

In addition to the T cell assays, an assay that utilizes transgenic animals can be used to verify that a nucleic acid construct functions (e.g., epitopes are processed and presented) when delivered *in vivo.* For measuring HLA-A2-restricted presentation, the nucleic acid construct in a mammalian expression vector (e.g., a plasmid) can be encapsulated in microparticles and introduced into HLA-A2 transgenic mice by a route such as intramuscular or subcutaneous injection. The construct may alternatively be administered without the microparticle delivery vehicle (e.g., the nucleic acid can be naked, associated with transfection facilitating agents, or in a recombinant virus). T cell responses are subsequently examined *in vitro* (Hedley et al., Nature Med. 4:365-68, 1998). Target and/or stimulator cells are T2A2 cells (T2 cells transfected with DNA encoding HLA-A2) or EL4.A2 cells (EL4 cells transfected with DNA encoding HLA-A2) pulsed with the A2 epitope being tested and T2A2 cells into which has been introduced a nucleic acid described herein. Parallel studies are performed using T2A2 cells pulsed with no peptide or with an irrelevant peptide and T2 cells that are untransfected. Alternatively, the target and/or stimulator cells can be infected with a recombinant virus (e.g., a vaccinia virus) that contains the nucleic acid construct or encodes one or more HPV proteins. In this way, HLA-A2 epitopes that are processed and presented *in vivo* following administration of a nucleic acid described herein are identified.

### Delivery of Nucleic Acid Compositions

Various methodologies can be employed to promote the delivery, uptake, and/or expression of a nucleic acid described herein in an appropriate cell. A nucleic acid can be delivered in a composition containing a pharmaceutically acceptable carrier such as saline, as a colloidal suspension, or as powder, with or without a diluent. The nucleic acid can be "naked" or administered together with a delivery system such as lipids, liposomes, microparticles (microspheres or microcapsules), gold particles, ISCOMS, nanoparticles, polymers, condensing agents, polysaccharides, polyamino acids, dendrimers, saponins, polyoxamers, QS21, adsorption enhancing materials, adjuvants, or fatty acids.

The nucleic acids can be administered, e.g., orally, intramuscularly, intravenously, intraarterially, intrathecally, intradermally, intraperitoneally, intranasally, intrapulmonarily, intraocularly, intravaginally, intracervically, intrarectally or subcutaneously. For example, they can be introduced into the gastrointestinal tract or the respiratory tract, e.g., by inhalation of a solution or powder containing microparticles that contain the nucleic acid. Alternatively, administration can be local (e.g., the cervix) or systemic. Administration can be a combination of systemic and non-systemic (e.g., an individual can receive a systemic administration as well as a cervical injection).

It is expected that a dosage of approximately 1 to 2000 µg of DNA, would be administered per kg of body weight per dose. Where the subject is an adult human, administration regimens can include administration (e.g., intradermal, intramuscular, intravenous, oral, or subcutaneous administration) of 10-9000 µg of a plasmid DNA e.g., 100 to 2000, or 500 to 1000 µg, of plasmid DNA (optionally contained in a microparticle) delivered intramuscularly, repeated 3-6 times.. For example, a dose of 100-400 µg DNA or a dose of 100-200 µg DNA (e.g., contained in a microparticle) repeated 3 times (e.g., at 1, 2, or 3 week intervals) can be administered to a patient. Dosage for a given patient can depends upon many factors, including the patient's size, general health, sex, body surface area, age, the particular compound to be administered, time and route of administration, and other drugs being administered concurrently. Determination of optimal dosage is well within the abilities of a pharmacologist.

A nucleic acid described herein can be administered alone or in combination with one or more additional therapies for the treatment of an HPV-mediated disease. Examples of such therapies include chemotherapy, radiation, and/or surgery.

A nucleic acid described herein can be administered alone or in combination with one or more compositions designed to enhance an immune response. Examples of compositions that can.be co-administered with the nucleic acid include an adjuvant (e.g., resiquimod, Aldara®, and/or imiquimod (see, e.g., WO 01/97795)), CpG oligonucleotides (see, e.g., U.S. Patent No. 6,239,116), a cytokine (e.g., IL-12 or GMCSF), a nucleic acid encoding cytokine, or a viral vector (e.g., using a prime-boost strategy). Nucleic acids containing CpG dinucleotides may activate lymphocytes in an individual and redirect the individual's immune response (e.g., from a Th2 to a Th1 response). A co-administered composition need not be administered at the same time as the pharmaceutical composition. For example, the composition can be co-administered within, for example, 1, 2, 3, 6, or 12 hours, or 1, 2, 3, 4, 5, 6, or 7 days (before or after) of the administration of the pharmaceutical composition.

A nucleic acid can be delivered according to the methods described herein with a microparticle. The term microparticle, as used herein, includes microspheres and microcapsules. Exemplary microparticles described in, e.g., U.S. Patent No. 5,783,567. The microparticle can contain the nucleic acid embedded in a polymeric matrix or enclosed in a hollow shell of polymer. Alternatively, the nucleic acid can be on the surface of the microparticle, associated with the microparticle, or in suspension with the microparticle. Microparticles can be formed, for example, as detailed in WO 95/24929, WO 01/36583, U.S. 6,270,795; U.S. 6,309,569 and U.S. 6,406,719.

Microparticles can act to maintain the integrity of the nucleic acid, e.g., by maintaining the encapsulated nucleic acid in a nondegraded state. Microparticles of an appropriate size or combination of sizes can optionally be used for pulsed delivery of the nucleic acid, for delivery at a specific site, and/or for delivery to a specific target cell population. Microparticles can optionally be less than 10 microns in diameter (e.g., for use in maximizing delivery of a nucleic acid into a subject's phagocytotic cells). Microparticles that are equal to or greater than 10 µM in diameter can also be used (e.g., for injection or implantation in a tissue, where they form a deposit, wherein, as the deposit breaks down, the nucleic acid or encoded polypeptide is released gradually over time and taken up by neighboring cells).

The polymeric matrix can be a biodegradable polymer such as poly-lactide-co-glycolide(PLG), polylactide, polyglycolide, polyanhydride, polyorthoester, polycaprolactone, polyphosphazene, proteinaceous polymer, polypeptide, polyester, or a naturally occurring polymer such as starch, alginate, chitosan, or gelatin.

The microparticle can optionally include one or more stabilizer compounds. A stabilizer compound is a compound that acts to protect the nucleic acid (e.g., to keep it supercoiled and/or protect it from degradation) during the production of a micropartilce and/or after *in vivo* delivery. The stabilizer compound can optionally remain associated with the nucleic acid after release from the polymeric delivery system. Examples of stabilizer compounds include, but are not limited to, tris(hydroxymethyl)aminomethane (TRIS), ethylenediaminetetraacetic acid (EDTA), a combination of TRIS and EDTA (TE), a carbohydrate (e.g., sucrose, lactose, dextran, trehalose, cyclodextrin, or dextran sulfate), a cationic compound (e.g., a cationic peptide), a pluronic (e.g., Pluronic® F-68 (Sigma-Aldrich Co., St. Louis, MO)), a lipid (e.g., hexadecyltrimethylammonium bromide), and/or a DNA-condensing agent. Preparation of microparticles containing stabilizer compounds is described in, e.g., WO 00/53161.

A lipid can be a charged lipid (e.g., a cationic lipid), an anionic lipid (e.g., PEG-DSPE, taurocholic acid, or phosphatidyl inositol), a zwitterionic lipid, or can have no charge. Examples of lipids include cetyltrimethylammonium and phospholipids (e.g., phosphatidylcholine). The microparticles can contain optionally more than one lipid and/or more than one type of lipid (e.g., those lipids present in lecithin lipid preparations) and may also include one or more stabilizer compounds as described above.

A microparticles can be used to enhance delivery of a nucleic acid into a subject's cells (e.g., phagocytotic cells). Alternatively, a biodegradable micrioparticle can be injected or implanted in a tissue, where it form a deposit. As the deposit breaks down, the nucleic acid is released gradually over time and is taken up by neighboring cells (including APCs) as a free nucleic acid.

The nucleic acids as used in methods described herein can also be administered to an individual using agents such as hydrogels, lipids, dendrimers, pluronics, liposomes, or Immune Stimulating Complexes (ISCOMS).

The nucleic acids described herein can be introduced in to a cell or a tissue of a subject by electroporation (see, e.g. U.S. Patent No. 5,462,520, U.S. Patent No. 5,993,434, and U.S. Patent No. 6,261,281). Electroporation can occur, for example, *ex vivo* or *in vivo.* In an exemplary method, electroporation is employed for administration of 100-200 µg of a nucleic acid (with microparticles) repeated three times at three week intervals. At one or more administrations, immediately following intramuscular injection of the nucleic acid-containing microparticles, electroporation (100V, 8 pulses, 20 msec/pulse, 1 sec pulse interval, unipolar, 0.5cm gap (200V/cm)) is applied at the injection site using an electrode with a 2 needle array and a ECM830 generator (Genetronics, San Diego, CA).

### Treatment and Prevention of HPV-Mediated Disease

A pharmaceutical composition containing a nucleic acid that encodes a polypeptide of the present invention can be used to treat or prevent CIN, an HPV-mediated disease. The composition can trigger an HPV-specific immune response and/or an innate immune response in the individual.

The ability of a nucleic acid described herein to elicit an immune response (e.g., a CTL response) can be assayed, for example, by a ⁵¹Cr release assay, by measuring cytokine expression (e.g., IL-12, gamma-interferon, or tumor necrosis factor alpha) and/or by using MHC tetramers (to detect the presence of antigen specific T cells). Assays such as ELISA, intracellular cytokine staining, or ELISPOT, can also be used to measure cytokine profiles attributable to T cell activation. T cell proliferation can be measured using assays such as ³H-thymidine uptake. Delayed type hypersensitivity can be measured with a standard skin test using a peptide encoded by a nucleic acid, the nucleic acid itself, or a pharmaceutical composition described herein. Immune responses can also be measured in, for example, macrophages, polymorphonuclear monocytes, natural killer cells, and/or B cells.

In some instances, it may be unnecessary, undesirable, and/or difficult to detect an immune response in an individual following a treatment described herein. Nonetheless, the effectiveness of the treatment can be evaluated by the ability of the administered pharmaceutical composition to treat and/or prevent CIN in a given individual or in a population of individuals to whom the composition is administered.

The uses and compositions applied in the uses described herein can be used to treat or prevent CIN associated with HPV types 16 and 18, as well as other HPV types including but not limited to HPV types 6, 11, 31, 33, 35, 39, 44, 45, 52, 53, 54, 56, 58, 61, 66, 67, 69, CP8304, CP141, MM4, MM7, and/or MM9. Conditions associated with specific HPV types include high grade cervical intraepithelial neoplasia (HPV 31, 33, 35, 39, 44, 45, 52, 53, 54, 56,58,61,66,67,69, CP8304, CP141, MM4, MM7, and MM9).

An HPV-mediated disease can be diagnosed by a clinician, for example, by an examination of an HPV-affected tissue. An example of an HPV-affected tissue is a tissue having an intraepithelial lesion, which may be caused by infection and replication of an HPV and/or by transforming events caused by expression of a particular HPV protein (e.g., E6 and/or E7). HPV-infected basal epithelial stem cells do not express viral proteins at high levels, and the viral proteins expressed in these cells are generally limited to the early proteins (e.g. E1, E2, E6, and E7). As these cells replicate, differentiate, and migrate toward the epithelial surface, they begin to express late viral proteins (e.g., L1 and L2) and viral replication is initiated. The differentiation of these cells triggers viral replication. In certain instances, basal cells lose the ability to suppress viral gene expression, leading to high levels of E6 and/or E7 epression in these undifferentiated stem cells. This can lead to a transforming event, the outgrowth of cells that overexpress viral oncoproteins (e.g., E6 and E7), and the onset of high grade cervical intraepithelial neoplasia. Host cell morphologies change as a consequence of the production and transformation.

Analysis of a cervical cytology sample (Pap test) permits the identification of cells having an altered morphology indicative of HPV infection (e.g., LSIL or CIN1) or a transforming pre-cancerous event (e.g. HSIL, CIN2, or CIN3). Histological diagnosis of a biopsy taken from tissues diagnosed cytologically as LSIL or HSIL renders a histological diagnosis of CIN1 or CIN2 and CIN3, respectively. It is difficult for pathologists to reliably distinguish between CIN2 and CIN3, so they are often considered together as CIN2/3. Thus, in many instances LSIL and CIN1 are considered as equivalent conditions and likewise, HSIL and CIN2/3 are considered as equivalent. Given the high spontaneous regression rate of LSIL (60-70%), the condition is generally monitored by consecutive Pap tests performed at 6-month intervals or by colposcopy followed by repeat Pap testing (Schiffman et al, Acta Cytol. 44:726-42, 2000). A small fraction of persistent LSIL (CIN1) progresses to HSIL (CIN2/3), and for this reason, persistent CIN1 is often treated by ablation or surgery. Striking changes in cell morphology, that include specific chromosomal and nuclear alterations are unique to HSIL and are reflective of this more serious, pre-cancerous state that is treated invasively with surgery or ablation. The occurrence of HSIL of the uterine cervix is highly correlated with the persistence of HPV residing in the cervical epithelium.

Individuals to be treated using the compositions applied in the uses described herein are less than 25 years of age. For example, a subject teated according to any of the uses described herein can have an age in the range of, e.g., 12-24, 12-20, 16-24, 16-20, 18-24 or 18-20. In addition to humans, mammals such as dogs, rabbits, rats, and mice may be administered the compositions described herein to treat an HPV-mediated disease (e.g., a model of a human disease) in the mammal

Eliminating or reducing the severity of an HPV-mediated disease such as high-grade intraepithelial neoplasia can result, for example, in resolution of a high-grade intraepithelial neoplasia and a subsequent clinical diagnosis of normal or low grade intraepithleial neoplasia. Eliminating a reducing CIN2/3 can result, for example, in the absence of CIN2/3 and the a diagnosis of normal or CIN1.

An individual with an HPV infection that has not progressed to CIN1 can be treated to prevent and/or reduce the chance of progression to CIN1, CIN2 or CIN3. In another example, an individual with LSIL (CIN 1) can be treated to present and/or reduce the chance of progression to HSIL. In another example, an individual with HSIL (CIN 2 or CIN 3) can be treated to prevent and/or reduce the chance of progression to carcinoma.

The diagnosis of CIN (as well as determining whether CIN has been eliminated or reduced in severity) is well known by medical practitioners, and include but are not limited to, visual inspection, a Pap test, cytology, histology, HPV testing (e.g., Hybrid Capture® or PCR), colposcopy, cervicography, optical imagine, anoscopy, endocervical curettage, loss of heterozygosity (LOH) analysis, measuring of viral load in cervical or vaginal secretion (e.g., a Pap sample). Hybrid Capture® (Diagene, Gaithersburg, MD) measures the presence of high and/or low risk HPV types present in a ThinPrep® Pap test sample. A positive reading indicates a minimally defined level of virus is present in the sample.

The use of a composition described herein can result in a suppression of viral infection. Suppression of viral infection may be indicated by any one or more of a number of parameters, including but not limited to, improvement of one or more symptoms of CIN and/or a detectable reduction in viral load (e.g., wherein a positive result from a hybrid capture assay resolves into a negative result after a treatment). Levels of viral nucleic acids can be assessed, e.g., by isolating nucleic acids from a patient sample and performing PCR analysis or blot analysis using virus specific primers (PCR analysis) or using a viral polynucleotide sequence as a probe (blot analysis). The PCR analysis can optionally be carried our to provide quantitative results. Viral infection can also be assessed by in situ hybridization with virus-specfic probes.

Another assay for detecting viral infection measures infectious units, such as infectious center assay (ICA). The extent or amount of viral particles can be measured from any infected area, such as an infected tissue or mucosal discharge.

When the sample is a liquid, viral titer can be calculated as a measure of the number or amount of virus or virus particles (e.g., infectious particles, plaque forming units, infectious doses, or median tissue culture infectious dose (TCID50)) per unit volume. In solid samples, such as a tissue sample, viral titer can be calculated in virus particles per unit weight. Reduction of viral infection can be indicated, for example, by comparing the measured viral titer (after treatment) to titer measured at an earlier time point or by comparing the measured titer to a standard titer (based, for example, on animal or clinical studies) that represents an untreated viral infection.

Suppression of viral infection can also be detected by measuring the elimination of cells in a diseased tissue of a subject that express at least one HPV protein. The elimination of such cells can be detected by methods generally used to detect CIN, for example, changes that occur in cytological samples, histological samples, or colposcopic impressions. Alternatively, Hybrid Capture® or PCR testing can be performed on the tissue of interest or on secretions or washes of the tissue.

### Diagnostics

Methods of typing HPV-mediated viruses are known in the art and include Southern blots, dot blots, measuring levels of gene expression, as in U.S. 6,355,424, for example or via polymerase chain reaction techniques as described, for example in U.S. 5,814,448.

Viral typing may also be accomplished by using a heteroduplex tracking assay (HTA). A HTA is a hybridization based method of determining the genetic relationship between two or more viral genomes. The basis of the method is that related DNA products co-amplified from divergent templates reanneal randomly to form heteroduplexes that migrate with reduced mobility in systems designed to separate molecules on the basis of size such as neutral polyacrilamide gels. Another method of identification of HPV genotypes is through the use of a line probe assay, a highly specific DNA hybridization test. Line probe assays for HPV are commercially available from Innogenetics (INNO-LiPA HPV Genotyping, Innogenetics N.V., Zwinjnaarde, Belgium). Another method of viral typing is by use of microarray or GeneChip technology. High density and spotted oligonucleotide arrays can be used to identify polymorphims of pathogens (see, for example, Kato-Maeda, et al., Cell Microbiol. 3(11):713-19 (2001)) and thus are a useful tool for identifying viral types and sub-types. GeneChip assays that are specific for HPV are commercially available from Norchip (PreTect® HPV-Proofer, NorChip AS, Klokkarstua, Norway).

The following are examples of the practice of the invention. They are not to be construed as limiting the scope of the invention in any way.

### Examples

### Example 1. Investigational Study of ZYC101a for the Treatment of HSIL of the Uterine Cervix

A study with ZYC101a for the treatment of HSIL of the uterine cervix was performed as a Phase 2, multi-center, international, double-blind, placebo-controlled, trial. ZYC101a is a formulation comprised of plasmid DNA encapsulated in biodegradable poly (D,L-lactide-co-glycolide) (PLG) microparticles. The ZYC101a plasmid encodes a polypeptide that includes HPV 16 and 18 coding sequences and was optimized for increased immunogenicity by inclusion of immunogenic regions of HPV16 E6 and E7 proteins and HPV18 E6 and E7 proteins. The ZYC101a plasmid, which is described in detail in WO 01/19408, encodes a polypeptide having the following amino acid sequence:
MAISGVPVLGFFIIAVLMSAQESWAAMFQDPQERPRKLPQLCTELLLRREVYDFAFRDLCIVY RDGNPYKISEYRHYCYSLYGTTLEQQYNKTLHEYMLDLQPETTDLYSYQAEPDRAHYNIVTF LLMGTLGIVCPICSQKPRRPYKLPDLCTELNTSLQDIEITCVYCKTVLELTEVFEFAFKSVYGDT LEKLTNTGLYNLLIRCLRCQKKATLQDIVLHLEPQNEIPVHTMLCMCCKCEARIAFQQLFLNTL SFVCPWC.

The randomized, double-blind, placebo-controlled study compared the effectiveness of 2 doses of ZYC101a (2 dose level:100 µg and 200 µg) to placebo in the treatment of HSIL of the uterine cervix. In order to be eligible for randomization into the trial, patients had HSIL (CIN II/III) (diagnosed by histology of tissue derived from colposcopically-directed punch biopsy), had a positive test result for HPV (as determined by hybrid capture or PCR testing of the Pap test sample), and had the diagnosis of CIN II/III confirmed by colposcopy between the time of biopsy and the first injection of ZYC101a. Approximately six months after entry into the study, all of the patients were treated with a LEEP procedure. A panel of blinded independent pathologists provided the histological diagnosis of tissue obtained at biopsy and at LEEP.

Subjects were randomized in a 1:1:1 ratio to receive ZYC101a 100µg, ZYC101a 200µg, or placebo.

### Selection and Timing of Dose for Each Patient

Sites were supplied with kits for reconstitution of the drug. ZYC101 a was supplied as lyophilized powder containing approximately 100 µg of DNA per vial. Each subject was to receive a single, IM (lateral quadriceps muscle) injection of study drug (100 µg or 200 µg of active drug or 1 mL of placebo) at weeks 0, 3, and 6. Placebo was sterile saline.

### Identity Of Investigational Product And Blinding

To maintain blinding, a research pharmacist or appropriate designee not associated with patient care, reconstituted and prepared the study drug solution for IM injection. Packaging was identical for all treatment assignments.

### Extent of Drug Exposure

Table 1 depicts the amount of drug exposure in patients. Patients received a range of 100 to 600 µg of ZYC101 a. The total expected amount of drug exposure in the 100 µg group was 300 µg. The total expected amount of drug exposure in the 200 µg group was 600 µg.

**Table 1: Extent of Drug Exposure**

| Total Drug Exposure (µg DNA) | Number of Patients Receiving the drug exposure |
|---|---|
| 600 | 54 |
| 400 | 1 |
| 300 | 51 |
| 200 | 1 |
| 100 | 2 |

### Demographic and other Baseline Characteristics

Patients were evenly distributed over all treatment groups with regard to various demographics: race, age, HLA-type, oral birth control usage, smoking and number of pregnancies.

Patients were evenly distributed over all treatment groups with regard to various baseline diagnostic characteristics: HPV type, cytology, colposcopy, and HC-II.

### Measurements of Treatment Compliance

The majority of patients enrolled in the study received all 3 planned injections. A total of 6 patients received fewer than 3 injections, including 2 patients in the placebo group, 1 patient in the 100 µg group, and 3 patients in the 200 µg group.

### Example 2: Efficacy and Safety Measurements Assessed and Flow Chart

### Efficacy

The efficacy measurement assessed during the study was histological rating of cervical tissue from LEEP (presence or absence of HSIL) as determined by a panel of independent pathologists (IPP) who were blinded as to whether patients received drug or placebo.

A subject who received at least one dose of study drug, underwent definitive therapy (LEEP) for HSIL, and who was monitored according to the protocol visit schedule for up to 6 months from the date of the first injection was considered to be a completed patient. A subject could withdraw from the trial voluntarily at any time. Furthermore, the Principal Investigator might judge, at any time, that it was in the subject's best interest to withdraw from the trial prematurely. These subjects proceeded to early LEEP. Assessed safety measurements included number of patients who progressed to early LEEP, reported adverse events, vital signs, physical examination and clinical laboratory testing.

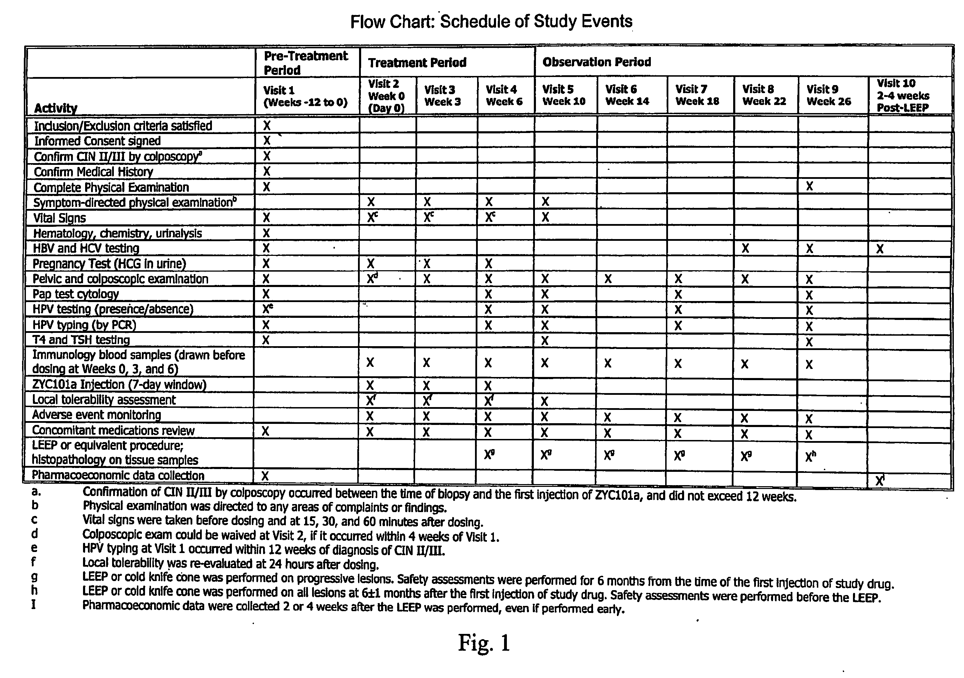

Fig. 1 depicts the schedule of study events. The study was divided into three periods: a pre-treatment period; a treatment period; and an observation period. Activities related to the study were monitored throughout the study in different periods. The following markers were evaluated throughout the study: immune response to HPV; Pap test cytology; HPV testing with Hybrid Capture-II and/or PCR; and presence of any colposcopically visualized lesion.

### Primary Efficacy Analysis

The primary efficacy endpoint was the histological diagnosis of cervical tissue from LEEP (presence or absence of HSIL) as determined by the IPP. A subject was said to have had "histological resolution" if the baseline biopsy was diagnosed for HSIL, and "LSIL" or "Normal" or "Atypical squamous epithelium, suggestive of HPV" was diagnosed for the tissue obtained during LEEP. Both the initial and final biopsy slides were collected and prepared by local pathology labs prior to being sent for independent review. Of the 161 patients enrolled, randomized, and dosed (and analyzed as the "safety population"), 127 were evaluable for the primary endpoint (mod ITT). To be eligible for evaluation of the primary endpoint, 2 criteria were needed: (1) diagnosis of HSIL (CIN2/3) by the local pathologist had to be confirmed by the IPP; and (2) LEEP tissue from the patient had to be available for diagnosis by the IPP.

If the diagnosis from each of two members of the IPP (reader 1 and reader 2) showed discordant interpretations for either initial or final histology slides, these slides then entered an adjudication process and were read by entire IPP (reader 1, reader 2, and reader 3). The adjudication process for a slide required that each member of the IPP physically meet and together either 1) reach agreement on an interpretation, or 2) agree to not reach an agreement. Table 2 depicts kappa values that were generated for each of the three pairs of readers. There was good agreement between each combination of pairs of pathologists participating in the IPP. Kappa values of 0.6 to 0.8 show substantial agreement and 0.8 to 1.0 represent almost perfect agreement for.inter-observer variability in the diagnosis of LEEP tissue (Stoler, et al., JAMA 287:2114-2119, 2002). The data indicated excellent agreement among readers and further supports the robustness of the results.

**Table 2: Inter-Observer Variability in Diagnosis of LEEP tissue**

| Reader Pair | Kappa |
|---|---|
| 1,2 | 0.77 |
| 2,3 | 0.84 |
| 1,3 | 0.76 |

Table 3 demonstrates that treatment of the <25 year old population resulted in a significant increase in the resolution rate for those patients in the ZYC101a treated groups versus placebo (P < 0.01). No statistically significant difference was observed between the 100 µg and 200 µg dose levels. For efficacy analyses, placebo was compared against the two active groups combined using a Fischer's exact test, and only if the p-value was lower than 5% was placebo compared against each of the active treatment groups separately. This stepwise procedure keeps the overall Type I error rate at 5% so that no further adjustment for multiple comparisons was necessary.

**Table 3: Proportion of Patients who have Histological Resolution of Lesions to non-HSIL or Normal at LEEP**

| | | | **Resolution** | |
|---|---|---|---|---|
| **Populations** | **Treatment Groups** | | **non-HSIL** **n(%)** | **normal** **n(%)** |
| **Mod ITT** | Placebo | n=41 | 11/41 (27) | 8/41 (20) |
| **n=127** | Combined | n=86 | 37/86 (43) p=0.11 | 22/86 (26) p=0.51 |
| | | | | |
| | Placebo | n=13 | 3 (23) | 1(8) |
| **<25 years old** | 100µg | n=12 | 8(67) p=0.05 | 4(33) |
| **n=43** | 200µg | n=18 | 13(72) p=0.01 | 7(39) |
| | Combined | n=30 | 21(70) p<0.01 | 11(37) p=0.07 |
| | | | | |
| | Placebo | n= 24 | 7(29) | 5 (21) |
| | | | | |
| | 100µg | n= 22 | 11(50) | 6 (27) |
| **<30 years old n=74** | 200µg | n=28 | 17(61) | 10 (36) |
| | Combined | n=50 | 28(56) p=0.05 | 16 (32) p = 0.4 |

In patients <25 yrs, the resolution rate to non-HSIL for patients on placebo was 23%. The proportion of patients who had histological resolution in the 100 µg group was 67% and in the 200 µg group was 72%. The proportion of patients in the combined ZYC101a treatment group was 70% (p<0.01).

In patients <25 yrs, the resolution rate to normal for patients on placebo was 8%. The rate for the 100 µg group was 33% and the 200 µg group was 39%. The proportion of patients in the combined ZYC101a treatment groups with resolution to normal was 37% (p=0.07).

### Safety

Adverse events (AE) that occurred on or after the day of the first dose of study drug and up to 26 weeks after the first dose of study drug were summarized. An AE was any untoward medical occurrence in a subject participating in the clinical study regardless of causality.

Standard clinical laboratory parameters, including hematology and chemistry were monitored during the study. Investigators determined clinically significant abnormalities and any changes of clinical concern. Clinical laboratory values were tagged as "Normal", "Low," or "High" if they were within, below or above their respective normal ranges.

### Adverse Events Analysis

Table 4 summarizes the frequency of patients reporting adverse events. Investigator terms for AEs (verbatim terms) were coded to preferred terms based on MedDRA Version 3.3. All events were listed by treatment, subject, and onset date. Incidence rates were summarized by event and treatment group. Overall incidence rates of subjects who experienced any AE were calculated by treatment group and presented in the same table. The intensity of AEs were defined by the following: Mild (transient symptoms that do not interfere with subject's daily activities and are acceptable); Moderate (marked symptoms that moderately interfere with subject's daily activities and are acceptable); and Severe (symptoms that interfere considerably with subject's daily activities and are unacceptable).

**Table 4: Frequency of Patients Reporting Adverse Events (5% combined treated group)**

| **Events** | **Placebo** **n=50** **(%)** | **100µg** **n=53** **(%)** | **200µg** **n=58** **(%)** | **Combined** **ZYC101a** **Treatment** **Groups** **n=111** **(%)** |
|---|---|---|---|---|
| Injection Site Pain¹ | 32.0 | 62.3 | 72.4 | 67.6 |
| Injection Site Erythema¹ | 6.0 | 15.1 | 24.1 | 19.8 |
| InjectionSite Induration¹ | 0.0 | 13.2 | 19.0 | 16.2 |
| Headache NOS² | 12.0 | 17.0 | 12.1 | 14.4 |
| Injection site reaction (NOS)¹ | 4.0 | 13.2 | 13.8 | 13.5 |
| Sore throat NOS | 6.0 | 13.2 | 13.8 | 13.5 |
| Nasopharangitis | 14.0 | 5.7 | 12.1 | 9.0 |
| Cough | 10.0 | 9.4 | 8.6 | 9.0 |
| Diarrhoea NOS | 6.0 | 9.4 | 8.6 | 9.0 |
| Fatigue | 4.0 | 9.4 | 6.9 | 8.1 |
| Nausea | 8.0 | 7.5 | 8.6 | 8.1 |
| Abdominal pain lower | 6.0 | 3.8 | 8.6 | 6.3 |
| Vaginosis fungal | 6.0 | 7.5 | 5.2 | 6.3 |
| Back Pain | 2.0 | 9.4 | 5.2 | 7.2 |
| Sinus Congestion | 4.0 | 7.5 | 5.2 | 6.3 |
| Vaginal discharge | 6.0 | 7.5 | 3.4 | 5.4 |
| Pain in Limb | 4. | 5.7 | 6.9 | 6.3 |
| Urinary Tract Infection | 12.0 | 3.8 | 6.9 | 5.4 |

| | | | | |
|---|---|---|---|---|
| ¹Local injections site adverse events ²Not otherwise specified | | | | |

Overall, ZYC101a was determined to be safe and well-tolerated. There was no evidence of systemic toxicity associated with the drug. As noted previously, there was an increase over placebo in the proportion of patients reporting injection site reactions in patients receiving ZYC101a. Those adverse events that appear to be associated with ZYC101a treatment were primarily limited to the injection site that included pain, erythema, induration, and nonspecific reactions. Local injection site reactions were either mild to moderate in severity and were treated with either no intervention or over the counter analgesics. There appeared to be a small dose effect in the number of patients reporting these events when comparing the 100µg and 200 µg groups. No changes were noted from baseline on physical exam. There were no clinically significant changes in laboratory values from baseline including those for blood chemistry, hematology, liver function, or urinalysis. There was no evidence of clinically significant changes from vaseline in thyroid activity. No drug related serious adverse events were reported.

### Safety Conclusions

The drug is safe and well tolerated with no evidence of systemic adverse events. Local injection site reactions, including pain, erythema, and induration were reported in patients receiving ZYC101a. These events were mild to moderate and resolved without intervention or over-the-counter analgesic.

### Example 3: Immune Response Analysis

The number of HPV specific T-cells was enumerated at each visit using gamma interferon ELISPOT analysis. HPV-specfic T-cells were detected in the blood at study entry for ∼50% of the patients. This number went up in the younger patients (<25 population). At each study visit, approximately 40% of the patients demonstrated a trend towards elevated HPV-specific T-cell responses.

### Example 4: Cytopathology Analysis

At each time point, (baseline and Weeks 6, 10, 18 and 26), the number of patients with listed cytopathology findings, based on a Thin Prep Pap test was summarized by treatment group.

The data in Table 5 demonstrate that an improvement in a cytological diagnosis of HSIL to non-HSIL (from baseline to time of LEEP) occurred at a higher frequency in the ZYC101a combined treatment group than in the placebo group. Concordance data suggests that the cytology will remain a useful monitoring tool when patients are treated with ZYC101a.

**Table 5: Cytological Improvement at Time of LEEP in Patients Diagnosed with HSIL Cytology at Time of Entry**

| Treatment Group | Percent of Patients with Cytologic Improvement¹ (%) | | |
|---|---|---|---|
| | (modITT) | (<25 years of age) | (<30 years of age) |
| Placebo n=41 | 5/23 (22) | 1/8 (13) | 4/13 (31) |
| Combined ZYC101a n=86 | 20/44 (45) (p=0.07)² | 8/12(67) (p=0.03)² | 11/22 (50) (p=0.5)² |

| | | | |
|---|---|---|---|
| ¹Improvement is defined as resolution of HSIL and a resulting cytological diagnosis of either norman, ASCUS, or LSIL ²As determined by comparison of placebo to treatment groups using the Fisher exact probability test. | | | |

### Example 5: Virology Analysis

A positive result on a specimen collected by Pap test for either of the tests (PCR, HC-II) was considered as "presence." Rates of subjects with absence of virus at the time of final LEEP measurement were calculated and compared by the Fisher's exact test. The relative light units (RLU)/cutoff values for each specimen were analyzed and the viral load was estimated from the ratio of relative light units to positive control values. Changes in viral load over time were examined.

The HPV subtype diagnosed at baseline by PCR was considered cleared if none or a different type of HPV was found by PCR at the LEEP visit.

### Hybrid Capture

HC-II is a commercially available kit (Digene, Gaithersburg, MD) that measures the presence of high risk HPV types present in the ThinPrep® Pap test sample. All Pap tests performed in this clinical study used the ThinPrep Pap test. The high risk HPV panel includes HPV 16, 18, 31, 33, 39, 45, 51, 52, 56, 59, 68. A positive reading indicates a defined level of virus is present in the sample. This is a positive/negative test and the results, when reported in this manner are not quantitative. The presence of virus is not meant to imply the presence of a lesion. Data from this analysis can be summarized in the following statements: most patients are HC-II positive at baseline and at LEEP; patients with a normal finding on histology are more likely to have a HC-II negative result that those who have a histology finding of HSIL or LSIL, histological resolution and a normal outcome does not always correspond to a HC-II negative result at LEEP.

Nearly all patients were HC-II positive at baseline. As expected, those patients who had a histological diagnosis of normal were more likely to be HPV HC-II negative than those who had a final LEEP diagnosis of LSIL.

### HPV type by PCR

The HPV type present in Pap test samples was evaluated throughout the trial by PCR analysis. Results at baseline from these typing studies were summarized as follows: HPV16 was detected in ∼50% patients, 35% of patients had HPV16 and no other detectable HPV viral type, HPV18 was detected in ∼7% patients, ∼40% of the patients did not have detectable HPV16 or HPV18 but had other HPV types. Table 6 details the specific HPV types detected in the CIN2/3patients at baseline.

**Table 6: HPV Subtypes Detected at Study Entry**

| **HPV Types at time of Entry Biopsy** | | **Frequency¹** |
|---|---|---|
| | n² | % |
| 16 | 63 | 42.0 |
| 58 | 13 | 8.7 |
| Novel | 12 | 8.0 |
| 52 | 10 | 6.7 |
| 31 | 9 | 6.0 |
| 18 | 9 | 6.0 |
| 53 | 5 | 3.3 |
| 56 | 4 | 2.7 |
| 33 | 3 | 2.0 |
| MM4 | 3 | 2.0 |
| 66 | 2 | 1.3 |
| 11 | 2 | 1.3 |
| 69 | 2 | 1.3 |
| 54 | 2 | 1.3 |
| CP141 | 2 | 1.3 |

| | | |
|---|---|---|
| ¹Virus types which were detected at a frequency of ≥ 1% are shown. Viruses that were detected at baseline but not above 1% included 67, 6, 35, 39, 44, 61, CP8304, MM7, and MM9. ²n are the number of subjects positive for each HPV type. The total is greater than 127, as some subjects were positive for more than one HPV type. | | |

Approximately 99% of the CIN2/3 patients, with test results, tested positive for HPV at baseline. Of the total CIN2/3 patient population, HPV 16 and/or 18 were detected at baseline in 56% of the patients, whereas neither HPV 16 nor 18 was detected in the remaining 44%. Similarly, within the CIN2/3 patient population less than 25 years of age, HPV 16 and/or 18 was detected at baseline in 55% of the patients, and neither HPV 16 nor 18 was detected in the remaining 45%. As demonstrated in Table 7, administration of ZYC101a elicited a significant resolution of CIN2/3 in patients under 25 years of age, irrespective of whether the subject was positive (resolution in 64% of patients in this group) or negative (resolution in 75% of patients in this group) for HPV 16 and/or 18. These findings demonstrate that
ZYC101a (which encodes a polypeptide containing segments of E6 and E7 proteins from HPV types 16 and 18) unexpectedly provides a therapeutic benefit for HPV-mediated disease caused by HPV types other than HPV 16 and/or 18.

**Table 7: Resolution of CIN2/3 in subjects <25 years of age**

| Virus | Placebo (%) | ZYC101a(%) |
|---|---|---|
| Subjects with HPV 16 and/or HPV18 | 22 | 64 |
| Subjects without HPV16 or HPV18* | 25 | 75 |

| | | |
|---|---|---|
| *99% of patients were positive by hybrid capture or PCR for HPV | | |

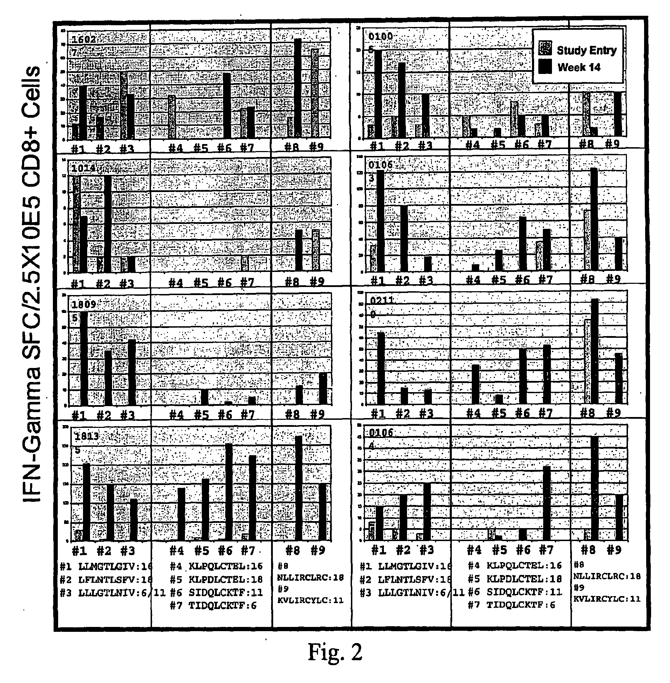

The *in vivo* cross-reactive immune response that was observed in CIN2/3 patients was further characterized by measuring the reactivity of T cells from patients treated with ZYC101a to peptides derived from differing HPV types. As depicted in Fig. 2, T cells from eight different ZYC101a-treated patients respond to peptides from HPV type 16 and HPV type 18. Seven of these eight also respond to peptides from HPV type 6 and HPV type 11, thus demonstrating that treatment of patients with ZYC101a can elicit T cells that recognize peptides derived from other HPV types, including HPV6 and HPV 11. HPV types 6 and 11, are the primary causative agents of anogenital warts. The experiments depicted in Fig. 2 were carried out using an ELISpot assay according to the manufacturer's instructions (R&D Systems). The T2/HLA-A2 cell line was used as an APC in the experiment, and was pre-pulsed with peptides prior to co-culture with enriched CD8 T cell populations from ZYC101a-treated patients. IFN-gamma production by the CD8 T cells was detected as a measure of T cell stimulation by the peptides presented on T2 cells.

### Example 6: Colposcopy Analysis

The proportions of patients with "no lesion" versus "at least one lesion" on colposcopy for placebo and each drug treatment group were recorded. The data suggest that overtime the number of lesions was reduced. It is predicted that the number and severity of highgrade lesions would be reduced over time in patients who exit the trial with resolution to non-HSIL.

### Example 7: Electroporation of ZYC101a in vivo

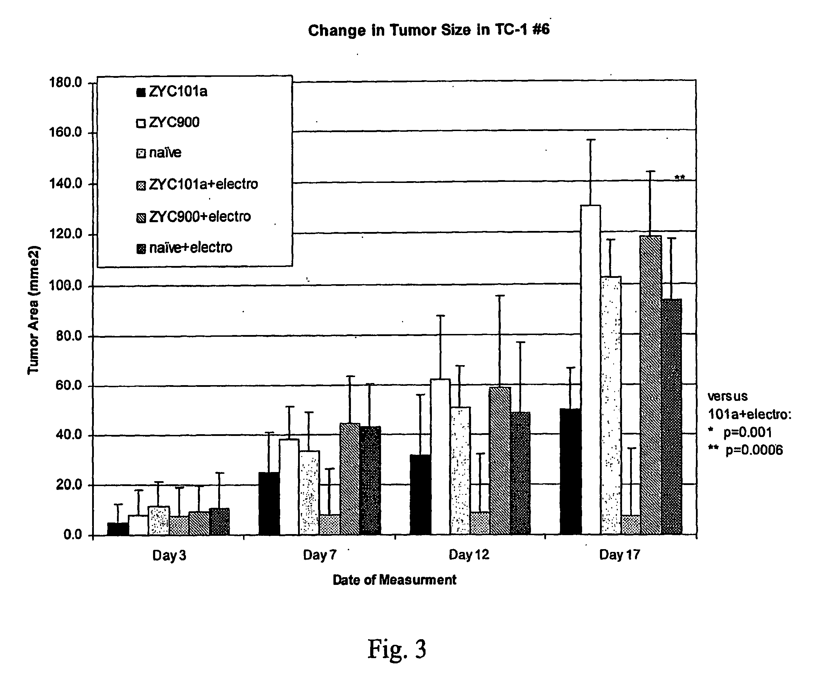

The effectiveness of administration of ZYC101a via electroporation was investigated in a murine model of HPV-mediated carcinoma. Mice were injected subcutaneously with the HPV16 E7-expressing tumor cell line, TC-1. In addition, mice were administered ZYC101a via intramuscular injection (both with and without concurrent electroporation) and tumor area was then measured over time. As controls, mice were administered ZYC900 (microparticle containing vector alone), given no treatment (naïve), or subjected to electroporation alone (naïve+electro). As demonstrated in Fig. 3, ZYC101a provided protection against tumor growth in the murine model of human disease, an effect that was enhanced when the microparticle/DNA ZYC101a composition was administered by electroporation.

### Other Embodiments

While the invention has been described in conjunction with the detailed description thereof, the foregoing description is intended to illustrate and not limit the scope of the invention, which is defined by the scope of the appended claims.

## Claims

1. Use of a nucleic acid comprising a nucleotide sequence that encodes a hybrid polypeptide for the preparation of a pharmaceutical composition for treating a cervical intraepithelial neoplasia (CIN) in a human identified as being less than 25 years of age, wherein the hybrid polypeptide comprises
at least one of the following segments of human papilloma virus (HPV)
strain 16 E6:
AMFQDPQERPRKLPQLCTEL,
LLRREVYDFAFRDLCIVYRDGNPY, or
KISEYRHYCYSLYGTTLEQQYNK;
at least one of the following segments of HPV strain 16 E7:
TLHEYMLDLQPETTDLYSY,
QAEPDRAHYNIVTF, or
LLMGTLGIVCPICSQKP;
at least one of the following segments of HPV strain 18 E6:
RRPYKLPDLCTELNTSLQDIEITCVYCKTVLELTEVFEFAFK, or
SVYGDTLEKLTNTGLYNLLIRCLRCQK; and
at least one of the following segments of HPV strain 18 E7:
KATLQDIVLHLEPQNEIPV,
HTMLCMCCKCEARI, or
AFQQLFLNTLSFVCPWC.

2. A nucleic acid comprising a nucleotide sequence that encodes a hybrid polypeptide for use in treating a cervical intraepithelial neoplasia (CIN) in a human identified as being less than 25 years of age, wherein the hybrid polypeptide comprises
at least one of the following segments of human papilloma virus (HPV) strain 16 E6:
AMFQDPQERPRKLPQLCTEL,
LLRREVYDFAFRDLCIVYRDGNPY, or
KISEYRHYCYSLYGTTLEQQYNK;
at least one of the following segments of HPV strain 16 E7:
TLHEYMLDLQPETTDLYSY,
QAEPDRAHYNIVTF, or
LLMGTLGIVCPICSQKP;
at least one of the following segments of HPV strain 18 E6:
RRPYKLPDLCTELNTSLQDIEITCVYCKTVLELTEVFEFAFK, or
SVYGDTLEKLTNTGLYNLLIRCLRCQK; and
at least one of the following segments of HPV strain 18 E7:
KATLQDIVLHLEPQNEIPV,
HTMLCMCCKCEARI, or
AFQQLFLNTLSFVCPWC.

3. The use of claim 1 or the nucleic acid of 2, wherein the hybrid polypeptide comprises the segments AMFQDPQERPRKLPQLCTEL,
LLRREVYDFAFRDLCIVYRDGNPY, KISEYRHYCYSLYGTTLEQQYNK,
TLHEYMLDLQPETTDLYSY, QAEPDRAHYNIVTF, LLMGTLGIVCPICSQKP,
RRPYKLPDLCTELNTSLQDIEITCVYCKTVLELTEVFEFAFK,
SVYGDTLEKLTNTGLYNLLIRCLRCQK, KATLQDIVLHLEPQNEIPV,
HTMLCMCCKCEARI, and AFQQLFLNTLSFVCPWC.

4. The use or the nucleic acid of any one of claims 1 to 3, wherein the hybrid polypeptide does not contain a sequence identical to the sequence of either full length, intact E6 or full length, intact E7 protein from HPV strain 16 or 18.

5. The use or the nucleic acid of any one of the preceding claims, wherein the hybrid polypeptide comprises a signal sequence.

6. The use or the nucleic acid of claim 5, wherein the signal sequence is the HLA-DRα leader sequence (MAISGVPVLGFFIIAVLMSAQESWA).

7. The use or the nucleic acid of any one of the preceding claims, wherein the hybrid polypeptide comprises the amino acid sequence
AMFQDPQERPRKLPQLCTELLLRREVYDFAFRDLCIVYRDGNPYKISEYRHYCYS LYGTTLEQQYNKTLHEYMLDLQPETTDLYSYQAEPDFLLMGTLGIV CPICSQKPRRPYKLPDLCTELNTSLQDIEITCVYCKTVLELTEVFEFAFKSVYGDTL EKLTNTGLYNLLIRCLRCQKKATLQDIVLHLEPQNEIPVHTMLCMCCKCEARIAF QQLFLNTLSFVCPWC.

8. The use or the nucleic acid of any one of the preceding claims, wherein the hybrid polypeptide comprises the amino acid sequence
MAISGVPVLGFFIIAVLMSAQESWAAMFQDPQERPRKLPQLCTELLLRREVYDFA FRDLCIVYRDGNPYKISEYRHYCYSLYGTTLEQQYNKTLHEYMLDLQPETTDLY SYQAEPDRAHYNIVTFLLMGTLGIVCPICSQKPRRPYKLPDLCTELNTSLQDIEITC VYCKTVLELTEVFEFAFKSVYGDTLEKLTNTGLYNLLIRCLRCQKKATLQDIVLH LEPQNEIPVHTMLCMCCKCEARIAFQQLFLNTLSFVCPWC.

9. The use or the nucleic acid of any one of the preceding claims, wherein the hybrid polypeptide consists of the amino acid sequence
MAISGVPVLGFFIIAVLMSAQESWAAMFQDPQERPRKLPQLCTELLLRREVYDFA FRDLCIVYRDGNPYKISEYRHYCYSLYGTTLEQQYNKTLHEYMLDLQPETTDLY SYQAEPDRAHYNIVTFLLMGTLGIVCPICSQKPRRPYKLPDLCTELNTSLQDIEITC VYCKTVLELTEVFEFAFKSVYGDTLEKLTNTGLYNLLIRCLRCQKKATLQDIVLH LEPQNEIPVHTMLCMCCKCEARIAFQQLFLNTLSFVCPWC.

10. The use or the nucleic acid of any one of the preceding claims, wherein the CIN is cervical intraepithelial neoplasia 1 (CIN1), cervical intraepithelial neoplasia 2 (CIN2), cervical intraepithelial neoplasia 3 (CIN3), or cervical intraepithelial neoplasia 2/3 (CIN2/3).

11. The use or the nucleic acid of any one of the preceding claims, wherein the nucleic acid comprises a plasmid vector.

12. The use or the nucleic acid of any one of claims 1 to 10, wherein the nucleic acid comprises a viral vector.

13. The use or the nucleic acid of any one of the preceding claims, wherein said pharmaceutical composition comprises a microparticle or wherein said nucleic acid is comprised in a pharmaceutical composition comprising a microparticle.

14. The use or the nucleic acid of any one of the preceding claims, wherein said pharmaceutical composition comprises a microparticle having the nucleic acid encapsulated therein or wherein said nucleic acid is comprised in a pharmaceutical composition comprising a microparticle having the nucleic acid encapsulated therein.

15. The use or the nucleic acid of claim 13 or 14, wherein the microparticle comprises a biodegradable poly (D,L-lactide co-glycolide).

16. The use or the nucleic acid of any one of claims 13-15, wherein the microparticle is less than 10 microns in diameter.

17. The use of any one of claims 1 or 3-16 or the nucleic acid of any one of claims 13-16, wherein the pharmaceutical composition comprises an adjuvant.

18. The use of any one of claims 1 or 3-17 or the nucleic acid of any one of claims 13-17, wherein the pharmaceutical composition is administered via injection.

19. The use or the nucleic acid of claim 18, wherein the injection is intramuscular, subcutaneous, or intracervical.

## Patentansprüche

1. Verwendung einer Nukleinsäure, umfassend eine Nukleotidsequenz, die für ein Hybridpolypeptid kodiert, zur Herstellung einer pharmazeutischen Zusammensetzung zur Behandlung einer zervikalen intraepithelialen Neoplasie (CIN) bei einem Menschen, bei dem ein Alter von unter 25 Jahren identifiziert wurde, wobei das Hybridpolypeptid
mindestens eines der folgenden Segmente des humanen Papillomavirus-(HPV-)Stamms 16 E6 umfasst:
AMFQDPQERPRKLPQLCTEL,
LLRREVYDFAFRDLCIVYRDGNPY oder
KISEYRHYCYSLYGTTLEQQYNK;
mindestens eines der folgenden Segmente des HPV-Stamms 16 E7:
TLHEYMLDLQPETTDLYSY,
QAEPDRAHYNIVTF oder
LLMGTLGIVCPICSQKP;
mindestens eines der folgenden Segmente des HPV-Stamms 18 E6:
RRPYKLPDLCTELNTSLQDIEITCVYCKTVLELTEVFEFAFK oder
SVYGDTLEKLTNTGLYNLLIRCLRCQK; und
mindestens eines der folgenden Segmente des HPV-Stamms 18 E7:
KATLQDIVLHLEPQNEIPV,
HTMLCMCCKCEARI oder
AFQQLFLNTLSFVCPWC.

2. Nukleinsäure, umfassend eine Nukleotidsequenz, die für ein Hybridpolypeptid kodiert, zur Verwendung bei der Behandlung einer zervikalen intraepithelialen Neoplasie (CIN) bei einem Menschen, bei dem ein Alter von unter 25 Jahren identifiziert wurde, wobei das Hybridpolypeptid
mindestens eines der folgenden Segmente des humanen Papillomavirus-(HPV-)Stamms 16 E6 umfasst:
AMFQDPQERPRKLPQLCTEL,
LLRREVYDFAFRDLCIVYRDGNPY oder
KISEYRHYCYSLYGTTLEQQYNK;
mindestens eines der folgenden Segmente des HPV-Stamms 16 E7:
TLHEYMLDLQPETTDLYSY,
QAEPDRAHYNIVTF oder
LLMGTLGIVCPICSQKP;
mindestens eines der folgenden Segmente des HPV-Stamms 18 E6:
RRPYKLPDLCTELNTSLQDIEITCVYCKTVLELTEVFEFAFK oder
SVYGDTLEKLTNTGLYNLLIRCLRCQK; und
mindestens eines der folgenden Segmente des HPV-Stamms 18 E7:
KATLQDIVLHLEPQNEIPV,
HTMLCMCCKCEARI oder
AFQQLFLNTLSFVCPWC.

3. Verwendung nach Anspruch 1 oder Nukleinsäure nach Anspruch 2, wobei das Hybridpolypeptid die Segmente AMFQDPQERPRKLPQLCTEL, LLRREVYDFAFRDLCIVYRDGNPY, KISEYRHYCYSLYGTTLEQQYNK, TLHEYMLDLQPETTDLYSY, QAEPDRAHYNIVTF, LLMGTLGIVCPICSQKP, RRPYKLPDLCTELNTSLQDIEITCVYCKTVLELTEVFEFAFK, SVYGDTLEKLTNTGLYNLLIRCLRCQK, KATLQDIVLHLEPQNEIPV, HTMLCMCCKCEARI und AFQQLFLNTLSFVCPWC umfasst.

4. Verwendung oder Nukleinsäure nach einem der Ansprüche 1 bis 3, wobei das Hybridpolypeptid keine Sequenz enthält, die entweder mit der Sequenz des intakten E6-Volllängenproteins oder des intakten E7-Volllängenproteins des HPV-Stamms 16 oder 18 identisch ist.

5. Verwendung oder Nukleinsäure nach einem der vorhergehenden Ansprüche, wobei das Hybridpolypeptid eine Signalsequenz umfasst.

6. Verwendung oder Nukleinsäure nach Anspruch 5, wobei die Signalsequenz die HLA-DRα Leadersequenz (MAISGVPVLGFFIIAVLMSAQESWA) ist.

7. Verwendung oder Nukleinsäure nach einem der vorhergehenden Ansprüche, wobei das Hybridpolypeptid die folgende Aminosäuresequenz umfasst:
AMFQDPQERPRKLPQLCTELLLRREVYDFAFRDLCIVYRDGNPYKISEYRHYC YSLYGTTLEQQYNKTLHEYMLDLQPETTDLYSYQAEPDRAHYNIVTFLLMGTLGI VCPICSQKPRRPYKLPDLCTELNTSLQDIEITCVYCKTVLELTEVFEFAFKSVY GDTLEKLTNTGLYNLLIRCLRCQKKATLQDIVLHLEPQNEIPVHTMLCMCCKCE ARIAF QQLFLNTLSFVCPWC.

8. Verwendung oder Nukleinsäure nach einem der vorhergehenden Ansprüche, wobei das Hybridpolypeptid die folgende Aminosäuresequenz umfasst:
MAISGVPVLGFFIIAVLMSAQESWAAMFQDPQERPRKLPQLCTELLLRREVYD FAFRDLCIVYRDGNPYKISEYRHYCYSLYGTTLEQQYNKTLHEYMLDLQPETTD LYSYQAEPDRAHYNIVTFLLMGTLGIVCPICSQKPRRPYKLPDLCTELNTSLQD IEITCVYCKTVLELTEVFEFAFKSVYGDTLEKLTNTGLYNLLIRCLRCQKKATLQD IVLHLEPQNEIPVHTMLCMCCKCEARIAFQQLFLNTLSFVCPWC.

9. Verwendung oder Nukleinsäure nach einem der vorhergehenden Ansprüche, wobei das Hybridpolypeptid aus der folgenden Aminosäuresequenz besteht:
MAISGVPVLGFFIIAVLMSAQESWAAMFQDPQERPRKLPQLCTELLLRREVYD FAFRDLCIVYRDGNPYKISEYRHYCYSLYGTTLEQQYNKTLHEYMLDLQPETT DLYSYQAEPDRAHYNIVTFLLMGTLGIVCPICSQKPRRPYKLPDLCTELNTSLQ DIEITCVYCKTVLELTEVFEFAFKSVYGDTLEKLTNTGLYNLLIRCLRCQKKATLQ DIVLHLEPQNEIPVHTMLCMCCKCEARIAFQQLFLNTLSFVCPWC.

10. Verwendung oder Nukleinsäure nach einem der vorhergehenden Ansprüche, wobei es sich bei der CIN um zervikale intraepitheliale Neoplasie 1 (CIN1), zervikale intraepitheliale Neoplasie 2 (CIN2), zervikale intraepitheliale Neoplasie 3 (CIN3) oder zervikale intraepitheliale Neoplasie 2/3 (CIN2/3) handelt.

11. Verwendung oder Nukleinsäure nach einem der vorhergehenden Ansprüche, wobei die Nukleinsäure einen Plasmidvektor umfasst.

12. Verwendung oder Nukleinsäure nach einem der Ansprüche 1 bis 10, wobei die Nukleinsäure einen viralen Vektor umfasst.

13. Verwendung oder Nukleinsäure nach einem der vorhergehenden Ansprüche, wobei besagte pharmazeutische Zusammensetzung einen Mikropartikel umfasst oder wobei besagte Nukleinsäure in einer pharmazeutischen Zusammensetzung enthalten ist, die einen Mikropartikel umfasst.

14. Verwendung oder Nukleinsäure nach einem der vorhergehenden Ansprüche, wobei besagte pharmazeutische Zusammensetzung einen Mikropartikel umfasst, der die Nukleinsäure darin einschließt, oder wobei besagte Nukleinsäure in einer pharmazeutischen Zusammensetzung enthalten ist, die einen Mikropartikel umfasst, der die Nukleinsäure darin einschließt.

15. Verwendung oder Nukleinsäure nach Anspruch 13 oder 14, wobei der Mikropartikel ein biologisch abbaubares Poly(D,L-lactid-co-glycolid) umfasst.

16. Verwendung oder Nukleinsäure nach einem der Ansprüche 13-15, wobei der Mikropartikel einen Durchmesser von weniger als 10 Mikrometer aufweist.

17. Verwendung nach einem der Ansprüche 1 oder 3-16 oder Nukleinsäure nach einem der Ansprüche 13-16, wobei die pharmazeutische Zusammensetzung ein Adjuvans umfasst.

18. Verwendung nach einem der Ansprüche 1 oder 3-17 oder Nukleinsäure nach einem der Ansprüche 13-17, wobei die pharmazeutische Zusammensetzung durch eine Injektion verabreicht wird.

19. Verwendung oder Nukleinsäure nach Anspruch 18, wobei die Injektion intramuskulär, subkutan oder intrazervikal erfolgt.

## Revendications

1. Utilisation d'un acide nucléique comprenant une séquence nucléotide qui encode un polypeptide hybride pour la préparation d'une composition pharmaceutique destinée à traiter une néoplasie intraépithéliale cervicale (CIN) chez un être humain identifié comme ayant moins de 25 ans, le polypeptide hybride comprenant
au moins un des segments suivants de la lignée de papillomavirus humain (HPV) 16 E6:
AMFQDPQERPRKLPQLCTEL,
LLRREVYDFAFRDLCIVYRDGNPY, ou
KISEYRHYCYSLYGTTLEQQYNK ;
au moins un des segments suivants de la lignée de HPV 16 E7 :
TLHEYMLDLQPETTDLYSY,
QAEPDRAHYNIVTF, ou
LLMGTLGIVCPICSQKP;
au moins un des segments suivants de la lignée de HPV 18 E6 :
RRPYKLPDLCTELNTSLQDIEITCVYCKTVLELTEVFEFAFK, ou
SVYGDTLEKLTNTGLYNLLIRCLRCQK; et
au moins un des segments suivants de la lignée de HPV 18 E7 :
KATLQDIVLHLEPQNEIPV,
HTMLCMCCKCEARI, ou
AFQQLFLNTLSFVCPWC.

2. Acide nucléique comprenant une séquence nucléotide qui encode un polypeptide hybride pour utilisation dans le traitement d'une néoplasie intraépithéliale cervicale (CIN) chez un être humain identifié comme ayant moins de 25 ans, le polypeptide hybride comprenant
au moins un des segments suivants de la lignée de papillomavirus humain (HPV) 16 E6:
AMFQDPQERPRKLPQLCTEL,
LLRREVYDFAFRDLCIVYRDGNPY, ou
KISEYRHYCYSLYGTTLEQQYNK;
au moins un des segments suivants de la lignée de HPV 16 E7:
TLHEYMLDLQPETTDLYSY,
QAEPDRAHYNIVTF, ou
LLMGTLGIVCPICSQKP;
au moins un des segments suivants de la lignée de HPV 18 E6:
RRPYKLPDLCTELNTSLQDIEITCVYCKTVLELTEVFEFAFK, ou
SVYGDTLEKLTNTGLYNLLIRCLRCQK; et
au moins un des segments suivants de la lignée de HPV 18 E7 :
KATLQDIVLHLEPQNEIPV,
HTMLCMCCKCEARI, ou
AFQQLFLNTLSFVCPWC.

3. Utilisation selon la revendication 1 ou de l'acide nucléique selon la revendication 2, le polypeptide hybride comprenant les segments AMFQDPQERPRKLPQLCTEL, LLRREVYDFAFRDLCIVYRDGNPY, KISEYRHYCYSLYGTTLEQQYNK, TLHEYMLDLQPETTDLYSY, QAEPDRAHYNIVTF, LLMGTLGIVCPICSQKP, RRPYKLPDLCTELNTSLQDIEITCVYCKTVLELTEVFEFAFK, SVYGDTLEKLTNTGLYNLLIRCLRCQK, KATLQDIVLHLEPQNEIPV, HTMLCMCCKCEARI, et AFQQLFLNTLSFVCPWC.

4. Utilisation ou l'acide nucléique selon l'une quelconque des revendications 1 à 3, le polypeptide hybride ne contenant pas une séquence identique ni à la séquence de protéine intacte E6 de longueur intégrale, ni à la séquence de protéine intacte E7 de longueur intégrale de la lignée HPV 16 ou 18.

5. Utilisation ou l'acide nucléique selon l'une quelconque des revendications précédentes, le polypeptide hybride comprenant une séquence de signaux.

6. Utilisation ou l'acide nucléique selon la revendication 5, la séquence de signaux étant la séquence de tête HLA-DRa (MAISGVPVLGFFIIAVLMSAQESWA).

7. Utilisation ou l'acide nucléique selon l'une quelconque des revendications précédentes, le polypeptide hybride comprenant la séquence d'acides aminés
AMFQDPQERPRKLPQLCTELLLRREVYDFAFRDLCIVYRDGNPYKISEYRH YCYSLYGTTLEQQYNKTLHEYMLDLQPETTDLYSYQAEPDRAHYNIVTFLL MGTLGIVCPICSQKPRRPYKLPDLCTELNTSLQDIEITCVYCKTVLELTEVFE FAFKSVYGDTLEKLTNTGLYNLLIRCLRCQKKATLQDIVLHLEPQNEIPVHTM LCMCCKCEARIAFQQLFLNTLSFVCPWC.

8. Utilisation ou l'acide nucléique selon l'une quelconque des revendications précédentes, le polypeptide hybride comprenant la séquence d'acides aminés
MAISGVPVLGFFIIAVLMSAQESWAAMFQDPQERPRKLPQLCTELLLRREV YDFAFRDLCIVYRDGNPYKISEYRHYCYSLYGTTLEQQYNKTLHEYMLDLQ PETTDLYSYQAEPDRAHYNIVTFLLMGTLGIVCPICSQKPRRPYKLPDLCTE LNTSLQDIEITCVYCKTVLELTEVFEFAFKSVYGDTLEKLTNTGLYNLLIRCLR CQKKATLQDIVLHLEPQNEIPVHTMLCMCCKCEARIAFQQLFLNTLSFVCPW C.

9. Utilisation ou l'acide nucléique selon l'une quelconque des revendications précédentes, le polypeptide hybride consistant en la séquence d'acides aminés
MAISGVPVLGFFIIAVLMSAQESWAAMFQDPQERPRKLPQLCTELLLRREV YDFAFRDLCIVYRDGNPYKISEYRHYCYSLYGTTLEQQYNKTLHEYMLDLQ PETTDLYSYQAEPDRAHYNIVTFLLMGTLGIVCPICSQKPRRPYKLPDLCTE LNTSLQDIEITCVYCKTVLELTEVFEFAFKSVYGDTLEKLTNTGLYNLLIRCLR CQKKATLQDIVLHLEPQNEIPVHTMLCMCCKCEARIAFQQLFLNTLSFVCPW C.

10. Utilisation ou l'acide nucléique selon l'une quelconque des revendications précédentes, l'on étant la néoplasie intraépithéliale cervicale 1 (CIN1), la néoplasie intraépithéliale cervicale 2 (CIN2), la néoplasie intraépithéliale cervicale 3 (CIN3), ou la néoplasie intraépithéliale cervicale 2/3 (CIN2/3).

11. Utilisation ou l'acide nucléique selon l'une quelconque des revendications précédentes, l'acide nucléique comprenant un vecteur plasmide.

12. Utilisation ou l'acide nucléique selon l'une quelconque des revendications 1 a 10, l'acide nucléique comprenant un vecteur viral.

13. Utilisation ou l'acide nucléique selon l'une quelconque des revendications précédentes, la composition pharmaceutique étant compris par une microparticule ou l'acide nucléique étant contenu dans une composition pharmaceutique, comprenant une microparticule.

14. Utilisation ou l'acide nucléique selon l'une quelconque des revendications précédentes, la composition pharmaceutique étant compris pas une microparticule, contenant l'acide nucléique quiest encapsulé dedans ou l'acide nucléique étant contenu dans une composition pharmaceutique, comprenant une microparticule contenant l'acide nucléique qui est encapsulé dedans.

15. Utilisation ou l'acide nucléique selon la revendication 13 ou 14, la microparticule comprenant un poly (D,L-lactide co-glycolide) biodégradable.

16. Utilisation ou l'acide nucléique selon l'une quelconque des revendications 13-15, la microparticule ayant un diamètre inférieur à 10 microns.

17. Utilisation selon l'une quelconque des revendications 1 ou 3 a 16 ou l'acide nucléique selon l'une quelconque des revendications 13 a 16, la composition pharmaceutique comprenant un adjuvant.

18. Utilisation selon l'une quelconque des revendications 1 ou 3-17 ou l'acide nucléique selon l'une quelconque des revendications 13-17 , la composition pharmaceutique étant administrée par injection.

19. Utilisation ou l'acide nucléique selon la revendication 18, l'injection étant intramusculaire, sous-cutanée ou intra-cervicale.
